(19) Europäisches Patentamt
European Patent Office
Office européen des brevets

(11) EP 1 323 823 A2

(12) # EUROPEAN PATENT APPLICATION

(43) Date of publication:
**02.07.2003 Bulletin 2003/27**

(51) Int Cl.[7]: **C12N 15/11**, C07K 16/18, C07K 16/42, A61K 38/17, C12Q 1/68, G01N 33/574

(21) Application number: **03002314.7**

(22) Date of filing: **02.07.1998**

(84) Designated Contracting States:
**AT BE CH CY DE DK ES FI FR GB GR IE IT LI LU MC NL PT SE**

(30) Priority: **03.07.1997 US 51704**
**03.07.1997 US 888088**
**19.05.1998 US 85983**
**19.05.1998 US 81338**
**17.06.1998 US 89899**
**17.06.1998 US 99005**

(62) Document number(s) of the earlier application(s) in accordance with Art. 76 EPC:
**98933133.5 / 0 998 559**

(71) Applicant: **Zymogenetics Inc**
**Seattle, WA 98102 (US)**

(72) Inventors:
- **Sheppard, Paul O.**
**Granite Falls, WA 98252 (US)**
- **Jaspers, Stephen R.**
**Edmonds, WA 98026 (US)**
- **Jelinek, Laura J.**
**Seattle, WA 98155 (US)**
- **Whitmore, Theodore E.**
**Redmond, WA 98052 (US)**

(74) Representative: **Schlich, George William et al**
**Mathys & Squire**
**100 Gray's Inn Road**
**London WC1X 8AL (GB)**

Remarks:
This application was filed on 03 - 02 - 2003 as a divisional application to the application mentioned under INID code 62.

(54) **Mammalian secretory peptide 9, antibodes against it and their use**

(57) Antibodies, antibody fragments or single chain antibodies that specifically bind to human and mouse orthologs of the Zsig-9 secreted polypeptide are provided, the antibodies are preferably monoclonal. Also provided are methods of using the antibodies of the invention as well as in-vitro methods of diagnosing cancer in human tissues or body fluids utilising the antibodies of the invention or antisense nucleic acid sequences that specifically bind to Zsig-9 polynucleotide sequences. The invention is of particular use in diagnosing cancers such as brain tumour, liver tumour, lung tumour, esophageal tumour, stomach tumour, colon tumour, rectal tumour, thyroid tumour, or lymphoma tumour.

EP 1 323 823 A2

SEQUENCE LISTING

(1) GENERAL INFORMATION

(i) APPLICANT: ZymoGenetics, Inc.
1201 Eastlake Avenue East
Seattle
WA
USA
98102

(ii) TITLE OF THE INVENTION: MAMMALIAN ZSIG9

(iii) NUMBER OF SEQUENCES: 24

(iv) CORRESPONDENCE ADDRESS:
(A) ADDRESSEE: ZymoGenetics Inc.
(B) STREET: 1201 Eastlake Ave East
(C) CITY: Seattle
(D) STATE: WA
(E) COUNTRY: USA
(F) ZIP: 98102

(v) COMPUTER READABLE FORM:
(A) MEDIUM TYPE: Diskette
(B) COMPUTER: IBM Compatible
(C) OPERATING SYSTEM: DOS
(D) SOFTWARE: FastSEQ for Windows Version 2.0

(vi) CURRENT APPLICATION DATA:
(A) APPLICATION NUMBER:
(B) FILING DATE:
(C) CLASSIFICATION:

(vii) PRIOR APPLICATION DATA:
(A) APPLICATION NUMBER:
(B) FILING DATE:

(viii) ATTORNEY/AGENT INFORMATION:
(A) NAME: Lunn, Paul G

(B) REGISTRATION NUMBER: 32,743
(C) REFERENCE/DOCKET NUMBER: 97-11PC

(ix) TELECOMMUNICATION INFORMATION:
(A) TELEPHONE: 206-442-6627
(B) TELEFAX: 206-442-6678
(C) TELEX:

(2) INFORMATION FOR SEQ ID NO:1:

(i) SEQUENCE CHARACTERISTICS:
(A) LENGTH: 649 base pairs
(B) TYPE: nucleic acid
(C) STRANDEDNESS: double
(D) TOPOLOGY: linear

(ii) MOLECULE TYPE: cDNA
(ix) FEATURE:

(A) NAME/KEY: Coding Sequence
(B) LOCATION: 104...354
(D) OTHER INFORMATION:

(xi) SEQUENCE DESCRIPTION: SEQ ID NO:1:

```
CGGCCCAAGG CTGGGGCCAA AGTGAAAGTC CAGCGGTCTN CCAGCGCTTG GGCCACGGCG    60
GCGGCCCTGG GACCAAAGGT GGAGCAACCC CGTTACCCTA AAR ATG AAA GGC TGG     115
                                                Met Lys Gly Trp
                                                 1

GGT TGG CTG GCC CTG CTT CTG GGG GCC CTG CTG GGA ACC GCC TGG GCT     163
Gly Trp Leu Ala Leu Leu Leu Gly Ala Leu Leu Gly Thr Ala Trp Ala
 5                  10                  15                  20

CGG AGG AGC CAG GAT CTC CAC TGT GGA GCA TGC AGG GCT CTG GTG GAT     211
Arg Arg Ser Gln Asp Leu His Cys Gly Ala Cys Arg Ala Leu Val Asp
                25                  30                  35

GAA CTA GAA TGG GAA ATT GCC CAG GTG GAC CCC AAG AAG ACC ATT CAG     259
Glu Leu Glu Trp Glu Ile Ala Gln Val Asp Pro Lys Lys Thr Ile Gln
            40                  45                  50
```

```
ATG GGA TCT TTC CGG ATC AAT CCA GAT GGC AGC CAG TCA GTG GTG GAG     307
Met Gly Ser Phe Arg Ile Asn Pro Asp Gly Ser Gln Ser Val Val Glu
        55                  60                  65

GTA ACT GTT ACT GTT CCC CCA AAC AAA GTA GCT CAC TCT GGC TTT GG AT   356
Val Thr Val Thr Val Pro Pro Asn Lys Val Ala His Ser Gly Phe Gly
    70                  75                  80

GAAATTCGAT TGCTTAAAAA GGACCTTGGT TTAATAGAAA TGAAGAAAAC AGACTCAGAA   416
AAAAGATTTG GCTCTGTCTC ATTTGGAAGA AGCTGCAGGC TTATTCCCCA TGCACTTGCT   476
TCCTGGCTGC AAACCTTAAT ACTTTGTTTN TGCTGTAGAA TTTGTTAGCA AACAGGGAGT   536
CCTGATCAGC ACCCTTNTCC ACATCCACAT GACTGGTTTT TAATGTAGCA CTGTGGTATA   596
CATGCAAACA TTCCGTTCAA AATCTGAGTC GGAGCTAAAA AAAAAAAAAA AAA          649
```

(2) INFORMATION FOR SEQ ID NO:2:

(i) SEQUENCE CHARACTERISTICS:
(A) LENGTH: 84 amino acids
(B) TYPE: amino acid
(C) STRANDEDNESS: single
(D) TOPOLOGY: linear

(ii) MOLECULE TYPE: protein
(v) FRAGMENT TYPE: internal

(xi) SEQUENCE DESCRIPTION: SEQ ID NO:2:

```
Met Lys Gly Trp Gly Trp Leu Ala Leu Leu Leu Gly Ala Leu Leu Gly
1               5                   10                  15
Thr Ala Trp Ala Arg Arg Ser Gln Asp Leu His Cys Gly Ala Cys Arg
            20                  25                  30
Ala Leu Val Asp Glu Leu Glu Trp Glu Ile Ala Gln Val Asp Pro Lys
        35                  40                  45
Lys Thr Ile Gln Met Gly Ser Phe Arg Ile Asn Pro Asp Gly Ser Gln
    50                  55                  60
Ser Val Val Glu Val Thr Val Thr Val Pro Pro Asn Lys Val Ala His
65                  70                  75                  80
Ser Gly Phe Gly
```

(2) INFORMATION FOR SEQ ID NO:3:

(i) SEQUENCE CHARACTERISTICS:
(A) LENGTH: 64 amino acids
(B) TYPE: amino acid

(C) STRANDEDNESS: single
(D) TOPOLOGY: linear

(xi) SEQUENCE DESCRIPTION: SEQ ID NO:3:

```
Arg Arg Ser Gln Asp Leu His Cys Gly Ala Cys Arg Ala Leu Val Asp
 1               5                  10                  15
Glu Leu Glu Trp Glu Ile Ala Gln Val Asp Pro Lys Lys Thr Ile Gln
            20                  25                  30
Met Gly Ser Phe Arg Ile Asn Pro Asp Gly Ser Gln Ser Val Val Glu
        35                  40                  45
Val Thr Val Thr Val Pro Pro Asn Lys Val Ala His Ser Gly Phe Gly
    50                  55                  60
```

(2) INFORMATION FOR SEQ ID NO:4:

(i) SEQUENCE CHARACTERISTICS:
(A) LENGTH: 62 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:4:

```
Ser Gln Asp Leu His Cys Gly Ala Cys Arg Ala Leu Val Asp Glu Leu
 1               5                  10                  15
Glu Trp Glu Ile Ala Gln Val Asp Pro Lys Lys Thr Ile Gln Met Gly
            20                  25                  30
Ser Phe Arg Ile Asn Pro Asp Gly Ser Gln Ser Val Val Glu Val Thr
        35                  40                  45
Val Thr Val Pro Pro Asn Lys Val Ala His Ser Gly Phe Gly
    50                  55                  60
```

(2) INFORMATION FOR SEQ ID NO:5:

(i) SEQUENCE CHARACTERISTICS:
(A) LENGTH: 25 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:5:

```
Ser Gln Asp Leu His Cys Gly Ala Cys Arg Ala Leu Val Asp Glu Leu
 1               5                  10                  15
Glu Trp Glu Ile Ala Gln Val Asp Pro
            20                  25
```

(2) INFORMATION FOR SEQ ID NO:6:

(i) SEQUENCE CHARACTERISTICS:
(A) LENGTH: 35 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:6:

```
Thr Ile Gln Met Gly Ser Phe Arg Ile Asn Pro Asp Gly Ser Gln Ser
 1               5                  10                  15
Val Val Glu Val Thr Val Thr Val Pro Pro Asn Lys Val Ala His Ser
            20                  25                  30
Gly Phe Gly
        35
```

(2) INFORMATION FOR SEQ ID NO:7:

(i) SEQUENCE CHARACTERISTICS:
(A) LENGTH: 417 base pairs
(B) TYPE: nucleic acid
(C) STRANDEDNESS: single
(D) TOPOLOGY: linear

(vi) ORIGINAL SOURCE:
(A) ORGANISM: EST192508

(xi) SEQUENCE DESCRIPTION: SEQ ID NO:7:

```
CTGGGGCAAA GTGAGAGTCC AGCGGTCTTC CAGCGCTTGG GCCACGGCGG CGGCCTGGGA     60
GCAGAGGTGG AGCGACCCCA TTACGCTAAA GATGAAAGGC TGGGGTTGGC TGGCCCTGCT    120
TCTGGGGGCC CTGCTGGGAA CCGCCTGGGC TCGGAGGAGC AGGGATCTCC ACTGTGGAGC    180
ATGCAGGGCT CTGGTGGATG AACTAGAATG GGAAATTGCC CAGGTGGACC CCAAGAAGAC    240
CATTCAGATG GGATCTTTCC GGATCAATCC AGATGGCAGC CAGTCAGTGG TNGAGGTAAC    300
```

TGTTACTGTT CCCCCAAACA AAGTAGCTCA CTCTGGCTTT NGATGAATTT CGATTTNTTT 360
AAAAAGGACC TTTGTTTTAT TAGGAATTGA AGAAAACAGA TTCAGAAAAA AGTTT
417

(2) INFORMATION FOR SEQ ID NO:8:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide
(vi) ORIGINAL SOURCE:
(A) ORGANISM: Flag

(xi) SEQUENCE DESCRIPTION: SEQ ID NO:8:

Asp Tyr Lys Asp Asp Asp Asp Lys Gly Ser
1 5 10

(2) INFORMATION FOR SEQ ID NO:9:

(i) SEQUENCE CHARACTERISTICS:
(A) LENGTH: 25 base pairs
(B) TYPE: nucleic acid
(C) STRANDEDNESS: single
(D) TOPOLOGY: linear

(vi) ORIGINAL SOURCE:
(A) ORGANISM: zc13654

(xi) SEQUENCE DESCRIPTION: SEQ ID NO:9:

GCGCGGATCC CGGAGGAGCC AGGAT 25

(2) INFORMATION FOR SEQ ID NO:10:

(i) SEQUENCE CHARACTERISTICS:
(A) LENGTH: 25 base pairs
(B) TYPE: nucleic acid
(C) STRANDEDNESS: single
(D) TOPOLOGY: linear

(iv) ANTISENSE: YES

(vi) ORIGINAL SOURCE:
(A) ORGANISM: ZC13.655

(xi) SEQUENCE DESCRIPTION: SEQ ID NO:10:

CGCGCTCGAG TCATCCAAAG CCAGA 25

(2) INFORMATION FOR SEQ ID NO:11:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: Other
(vi) ORIGINAL SOURCE:
(A) ORGANISM: ZC13656

(xi) SEQUENCE DESCRIPTION: SEQ ID NO:11:

GCGCGAATTC ATGAAAGGCT GGGGT 25

(2) INFORMATION FOR SEQ ID NO:12:

(i) SEQUENCE CHARACTERISTICS:
(A) LENGTH: 25 base pairs
(B) TYPE: nucleic acid
(C) STRANDEDNESS: single
(D) TOPOLOGY: linear

(iv) ANTISENSE: YES
(vi) ORIGINAL SOURCE:
(A) ORGANISM: ZC13657

(xi) SEQUENCE DESCRIPTION: SEQ ID NO:12:

CGCGGGATCC TCCAAAGCCA GAGTG 25

(2) INFORMATION FOR SEQ ID NO:13:

(i) SEQUENCE CHARACTERISTICS:
(A) LENGTH: 40 base pairs
(B) TYPE: nucleic acid
(C) STRANDEDNESS: single

(D) TOPOLOGY: linear

(iv) ANTISENSE: YES
(vi) ORIGINAL SOURCE:
(A) ORGANISM: ZC11.666

(xi) SEQUENCE DESCRIPTION: SEQ ID NO:13:

TTCATCCACC AGAGCCCTGC ATGCTCCACA GTGGAGATCC 40

(2) INFORMATION FOR SEQ ID NO:14:

(i) SEQUENCE CHARACTERISTICS:
(A) LENGTH: 18 base pairs
(B) TYPE: nucleic acid
(C) STRANDEDNESS: single
(D) TOPOLOGY: linear

(vi) ORIGINAL SOURCE:
(A) ORGANISM: zc 14.477

(xi) SEQUENCE DESCRIPTION: SEQ ID NO:14:

GGGCTCTGGT GGATGAAC 18

(2) INFORMATION FOR SEQ ID NO:15:

(i) SEQUENCE CHARACTERISTICS:
(A) LENGTH: 18 base pairs
(B) TYPE: nucleic acid
(C) STRANDEDNESS: single
(D) TOPOLOGY: linear

(iv) ANTISENSE: YES
(vi) ORIGINAL SOURCE:
(A) ORGANISM: ZC 14.489

(xi) SEQUENCE DESCRIPTION: SEQ ID NO:15:

TACCTCCACC ACTGACTG 18

(2) INFORMATION FOR SEQ ID NO:16:

(i) SEQUENCE CHARACTERISTICS:
(A) LENGTH: 806 base pairs

(B) TYPE: nucleic acid
(C) STRANDEDNESS: single
(D) TOPOLOGY: linear

(ii) MOLECULE TYPE: cDNA
(ix) FEATURE:

(A) NAME/KEY: Coding Sequence
(B) LOCATION: 104...649
(D) OTHER INFORMATION:

(xi) SEQUENCE DESCRIPTION: SEQ ID NO:16:

```
CGGCCCAAGG CTGGGGCCAA AGTGAAAGTC CAGCGGTCTN CCAGCGCTTG GGCCACGGCG      60
GCGGCCCTGG GACCAAAGGT GGAGCAACCC CGTTACCCTA AAR ATG AAA GGC TGG       115
                                                Met Lys Gly Trp
                                                 1

GGT TGG CTG GCC CTG CTT CTG GGG GCC CTG CTG GGA ACC GCC TGG GCT       163
Gly Trp Leu Ala Leu Leu Leu Gly Ala Leu Leu Gly Thr Ala Trp Ala
 5                  10                  15                  20

CGG AGG AGC CAG GAT CTC CAC TGT GGA GCA TGC AGG GCT CTG GTG GAT       211
Arg Arg Ser Gln Asp Leu His Cys Gly Ala Cys Arg Ala Leu Val Asp
                25                  30                  35

GAA CTA GAA TGG GAA ATT GCC CAG GTG GAC CCC AAG AAG ACC ATT CAG       259
Glu Leu Glu Trp Glu Ile Ala Gln Val Asp Pro Lys Lys Thr Ile Gln
            40                  45                  50

ATG GGA TCT TTC CGG ATC AAT CCA GAT GGC AGC CAG TCA GTG GTG GAG       307
Met Gly Ser Phe Arg Ile Asn Pro Asp Gly Ser Gln Ser Val Val Glu
        55                  60                  65

GTG CCT TAT GCC CGC TCA GAG GCC CAC CTC ACA GAG CTG CTG GAG GAG       355
Val Pro Tyr Ala Arg Ser Glu Ala His Leu Thr Glu Leu Leu Glu Glu
    70                  75                  80

ATA TGT GAC CGG ATG AAG GAG TAT GGG GAA CAG ATT GAT CCT TCC ACC       403
Ile Cys Asp Arg Met Lys Glu Tyr Gly Glu Gln Ile Asp Pro Ser Thr
85                  90                  95                  100

CAT CGC AAG AAC TAC GTA CGT GTA GTG GGC CGG AAT GGA GAA TCC AGT       451
His Arg Lys Asn Tyr Val Arg Val Val Gly Arg Asn Gly Glu Ser Ser
                105                 110                 115
```

```
GAA CTG GAC CTA CAA GGC ATC CGA ATC GAC TCA GAT ATT AGC GGC ACC      499
Glu Leu Asp Leu Gln Gly Ile Arg Ile Asp Ser Asp Ile Ser Gly Thr
            120                 125                 130

CTC AAG TTT GCG TGT GAG AGC ATT GTG GAG GAA TAC GAG GAT GAA CTC      547
Leu Lys Phe Ala Cys Glu Ser Ile Val Glu Glu Tyr Glu Asp Glu Leu
        135                 140                 145

ATT GAA TTC TTT TCC CGA GAG GCT GAC AAT GTT AAA GAC AAA CTT TGC      595
Ile Glu Phe Phe Ser Arg Glu Ala Asp Asn Val Lys Asp Lys Leu Cys
    150                 155                 160

AGT AAG CGA ACA GAT CTT TGT GAC CAT GCC CTG CAC ATA TCG CAT GAT      643
Ser Lys Arg Thr Asp Leu Cys Asp His Ala Leu His Ile Ser His Asp
165                 170                 175                 180

GAG CTA TGAACCACTG GAGCAGCCCA CACTGGCTTG ATGGATCACC CCCAGGAGGG GA   701
Glu Leu

AAATGGTGGC AATGCCTTTT ATATATTATG TTTTTACTGA AATTAACTGA AAAAATATGA    761
AACCAAAAGT AAAAAAAAAA AAAAAAAGAG AGAGAGAGAG AACTA                     806
```

(2) INFORMATION FOR SEQ ID NO:17:

(i) SEQUENCE CHARACTERISTICS:
(A) LENGTH: 182 amino acids
(B) TYPE: amino acid
(C) STRANDEDNESS: single
(D) TOPOLOGY: linear

(ii) MOLECULE TYPE: protein
(v) FRAGMENT TYPE: internal

(xi) SEQUENCE DESCRIPTION: SEQ ID NO:17:

```
Met Lys Gly Trp Gly Trp Leu Ala Leu Leu Leu Gly Ala Leu Leu Gly
 1               5                  10                  15
Thr Ala Trp Ala Arg Arg Ser Gln Asp Leu His Cys Gly Ala Cys Arg
            20                  25                  30
Ala Leu Val Asp Glu Leu Glu Trp Glu Ile Ala Gln Val Asp Pro Lys
        35                  40                  45
Lys Thr Ile Gln Met Gly Ser Phe Arg Ile Asn Pro Asp Gly Ser Gln
    50                  55                  60
```

```
Ser Val Val Glu Val Pro Tyr Ala Arg Ser Glu Ala His Leu Thr Glu
65                  70                  75                  80
Leu Leu Glu Glu Ile Cys Asp Arg Met Lys Glu Tyr Gly Glu Gln Ile
                85                  90                  95
Asp Pro Ser Thr His Arg Lys Asn Tyr Val Arg Val Val Gly Arg Asn
            100                 105                 110
Gly Glu Ser Ser Glu Leu Asp Leu Gln Gly Ile Arg Ile Asp Ser Asp
        115                 120                 125
Ile Ser Gly Thr Leu Lys Phe Ala Cys Glu Ser Ile Val Glu Glu Tyr
    130                 135                 140
Glu Asp Glu Leu Ile Glu Phe Phe Ser Arg Glu Ala Asp Asn Val Lys
145                 150                 155                 160
Asp Lys Leu Cys Ser Lys Arg Thr Asp Leu Cys Asp His Ala Leu His
                165                 170                 175
Ile Ser His Asp Glu Leu
            180
```

(2) INFORMATION FOR SEQ ID NO:18:

(i) SEQUENCE CHARACTERISTICS:
(A) LENGTH: 1069 base pairs
(B) TYPE: nucleic acid
(C) STRANDEDNESS: single
(D) TOPOLOGY: linear

(ii) MOLECULE TYPE: cDNA
(ix) FEATURE:

(A) NAME/KEY: Coding Sequence
(B) LOCATION: 358...903
(D) OTHER INFORMATION:

(xi) SEQUENCE DESCRIPTION: SEQ ID NO:18:

```
GAATTCGGCA CGAGGGGGGT CCTCGCTGCC TCGGAGGCGC TCCTAAAGCT GCCTGCTCGC    60
GCGAGAGTTT GGAGGGGCGG GCTTAGGGTC AGTTTCGGTG GGGGGCTCGC ACGGGACCCT   120
CAGATCTCCG CTTAGGTGCC TAGTTAAGTG CGGGAAGCTG GGCCAGGCGG TCACTGGCCA   180
CCCTGAACCT GGCGGGAGCC GGAGCGCTCT GGAGAAGCCG GGACAGCCCC GTTTTTCCCA   240
GCCAGCTGCT AGGGTTGGGA CCCACAGAAA ACAAAGTGAG AGTCCGGCTG CTTTCCAGAG   300
CCTGGGCCAC GGCGGCGGCC GTGGGAGCAG AGGTGGAGCG ACCCTGTTAC ACTAAAG ATG  360
                                                               Met
                                                                1
```

AAA GGC TGG GGT TGG CTA GCC CTA CTT TTG GGG GTC CTG CTG GGA ACT 408
Lys Gly Trp Gly Trp Leu Ala Leu Leu Leu Gly Val Leu Leu Gly Thr
5 10 15

GCC TGG GCT CGA AGG AGC CAA GAT CTA CAC TGT GGA GCT TGC AGG GCT 456
Ala Trp Ala Arg Arg Ser Gln Asp Leu His Cys Gly Ala Cys Arg Ala
20 25 30

CTG GTG GAT GAA TTA GAG TGG GAA ATT GCC CGC GTG GAC CCC AAG AAG 504
Leu Val Asp Glu Leu Glu Trp Glu Ile Ala Arg Val Asp Pro Lys Lys
35 40 45

ACC ATT CAG ATG GGA TCC TTC CGA ATC AAT CCA GAT GGC AGC CAG TCA 552
Thr Ile Gln Met Gly Ser Phe Arg Ile Asn Pro Asp Gly Ser Gln Ser
50 55 60 65

GTT GTG GAG GTA CCT TAT GCC CGC TCA GAG GCC CAC CTC ACA GAG TTG 600
Val Val Glu Val Pro Tyr Ala Arg Ser Glu Ala His Leu Thr Glu Leu
70 75 80

CTT GAG GAG GTG TGT GAC CGA ATG AAG GAG TAC GGG GAA CAG ATT GAC 648
Leu Glu Glu Val Cys Asp Arg Met Lys Glu Tyr Gly Glu Gln Ile Asp
85 90 95

CCT TCT ACC CAC CGC AAG AAC TAC GTA CGC GTC GTG AGC CGG AAT GGA 696
Pro Ser Thr His Arg Lys Asn Tyr Val Arg Val Val Ser Arg Asn Gly
100 105 110

GAA TCC AGT GAA CTA GAC TTA CAG GGC ATC CGA ATT GAC TCA GAT ATC 744
Glu Ser Ser Glu Leu Asp Leu Gln Gly Ile Arg Ile Asp Ser Asp Ile
115 120 125

AGC GGC ACC CTC AAG TTT GCG TGT GAG AGC ATT GTG GAA GAA TAC GAG 792
Ser Gly Thr Leu Lys Phe Ala Cys Glu Ser Ile Val Glu Glu Tyr Glu
130 135 140 145

GAT GAG CTT ATC GAA TTC TTC TCC AGA GAG GCT GAC AAC GTT AAA GAC 840
Asp Glu Leu Ile Glu Phe Phe Ser Arg Glu Ala Asp Asn Val Lys Asp
150 155 160

AAA CTT TGC AGT AAG CGG ACA GAT CTA TGT GAC CAT GCC CTG CAC AGA 888
Lys Leu Cys Ser Lys Arg Thr Asp Leu Cys Asp His Ala Leu His Arg
165 170 175

TCT CAC GAT GAG CTA TGAATCACTG GAGCAAGCAG CCTACACCAA ACGTGATGGA A 944
Ser His Asp Glu Leu
180

CACCCCCAGG AGGGGAAGAT GGCAGCATTG CCTTTTATAT TACGTTTTTA TGGAAATGAA 1004
CTGAAAAAAA CTCTTGAAAC CGAAAGTAAA AAAAAAAAAA AAAAAAAAAA AATTTCCGCG 1064
GCCGC 1069

(2) INFORMATION FOR SEQ ID NO:19:

(i) SEQUENCE CHARACTERISTICS:
(A) LENGTH: 182 amino acids
(B) TYPE: amino acid
(C) STRANDEDNESS: single
(D) TOPOLOGY: linear

(ii) MOLECULE TYPE: protein
(v) FRAGMENT TYPE: internal

(xi) SEQUENCE DESCRIPTION: SEQ ID NO:19:

Met Lys Gly Trp Gly Trp Leu Ala Leu Leu Leu Gly Val Leu Leu Gly
1 5 10 15
Thr Ala Trp Ala Arg Arg Ser Gln Asp Leu His Cys Gly Ala Cys Arg
20 25 30
Ala Leu Val Asp Glu Leu Glu Trp Glu Ile Ala Arg Val Asp Pro Lys
35 40 45
Lys Thr Ile Gln Met Gly Ser Phe Arg Ile Asn Pro Asp Gly Ser Gln
50 55 60
Ser Val Val Glu Val Pro Tyr Ala Arg Ser Glu Ala His Leu Thr Glu
65 70 75 80
Leu Leu Glu Glu Val Cys Asp Arg Met Lys Glu Tyr Gly Glu Gln Ile
85 90 95
Asp Pro Ser Thr His Arg Lys Asn Tyr Val Arg Val Val Ser Arg Asn
100 105 110
Gly Glu Ser Ser Glu Leu Asp Leu Gln Gly Ile Arg Ile Asp Ser Asp
115 120 125
Ile Ser Gly Thr Leu Lys Phe Ala Cys Glu Ser Ile Val Glu Glu Tyr
130 135 140
Glu Asp Glu Leu Ile Glu Phe Phe Ser Arg Glu Ala Asp Asn Val Lys
145 150 155 160
Asp Lys Leu Cys Ser Lys Arg Thr Asp Leu Cys Asp His Ala Leu His
165 170 175
Arg Ser His Asp Glu Leu
180

(2) INFORMATION FOR SEQ ID NO:20:

(i) SEQUENCE CHARACTERISTICS:
(A) LENGTH: 162 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:20:

```
Arg Arg Ser Gln Asp Leu His Cys Gly Ala Cys Arg Ala Leu Val Asp
 1               5                  10                  15
Glu Leu Glu Trp Glu Ile Ala Gln Val Asp Pro Lys Lys Thr Ile Gln
            20                  25                  30
Met Gly Ser Phe Arg Ile Asn Pro Asp Gly Ser Gln Ser Val Val Glu
        35                  40                  45
Val Pro Tyr Ala Arg Ser Glu Ala His Leu Thr Glu Leu Leu Glu Glu
    50                  55                  60
Ile Cys Asp Arg Met Lys Glu Tyr Gly Glu Gln Ile Asp Pro Ser Thr
65                  70                  75                  80
His Arg Lys Asn Tyr Val Arg Val Val Gly Arg Asn Gly Glu Ser Ser
                85                  90                  95
Glu Leu Asp Leu Gln Gly Ile Arg Ile Asp Ser Asp Ile Ser Gly Thr
            100                 105                 110
Leu Lys Phe Ala Cys Glu Ser Ile Val Glu Glu Tyr Glu Asp Glu Leu
        115                 120                 125
Ile Glu Phe Phe Ser Arg Glu Ala Asp Asn Val Lys Asp Lys Leu Cys
    130                 135                 140
Ser Lys Arg Thr Asp Leu Cys Asp His Ala Leu His Ile Ser His Asp
145                 150                 155                 160
Glu Leu
```

(2) INFORMATION FOR SEQ ID NO:21:

(i) SEQUENCE CHARACTERISTICS:
(A) LENGTH: 162 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:21:

Arg Arg Ser Gln Asp Leu His Cys Gly Ala Cys Arg Ala Leu Val Asp
1 5 10 15
Glu Leu Glu Trp Glu Ile Ala Arg Val Asp Pro Lys Lys Thr Ile Gln
20 25 30
Met Gly Ser Phe Arg Ile Asn Pro Asp Gly Ser Gln Ser Val Val Glu
35 40 45
Val Pro Tyr Ala Arg Ser Glu Ala His Leu Thr Glu Leu Leu Glu Glu
50 55 60
Val Cys Asp Arg Met Lys Glu Tyr Gly Glu Gln Ile Asp Pro Ser Thr
65 70 75 80
His Arg Lys Asn Tyr Val Arg Val Val Ser Arg Asn Gly Glu Ser Ser
85 90 95
Glu Leu Asp Leu Gln Gly Ile Arg Ile Asp Ser Asp Ile Ser Gly Thr
100 105 110
Leu Lys Phe Ala Cys Glu Ser Ile Val Glu Glu Tyr Glu Asp Glu Leu
115 120 125
Ile Glu Phe Phe Ser Arg Glu Ala Asp Asn Val Lys Asp Lys Leu Cys
130 135 140
Ser Lys Arg Thr Asp Leu Cys Asp His Ala Leu His Arg Ser His Asp
145 150 155 160
Glu Leu

(2) INFORMATION FOR SEQ ID NO:22:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: Other

(xi) SEQUENCE DESCRIPTION: SEQ ID NO:22:

TCGCGCGAGA GTTTGGAG 18

(2) INFORMATION FOR SEQ ID NO:23:

(i) SEQUENCE CHARACTERISTICS:
(A) LENGTH: 18 base pairs
(B) TYPE: nucleic acid

(C) STRANDEDNESS: single
(D) TOPOLOGY: linear

(ii) MOLECULE TYPE: Other

(xi) SEQUENCE DESCRIPTION: SEQ ID NO:23:

CCCAGCTTCC CGCACTTA 18

(2) INFORMATION FOR SEQ ID NO:24:

(i) SEQUENCE CHARACTERISTICS:
(A) LENGTH: 35 amino acids
(B) TYPE: amino acid
(C) STRANDEDNESS: single
(D) TOPOLOGY: linear

(xi) SEQUENCE DESCRIPTION: SEQ ID NO:24:

```
Arg Arg Ser Gln Asp Leu His Cys Gly Ala Cys Arg Ala Leu Val Asp
 1               5                  10                  15
Glu Leu Glu Trp Glu Ile Ala Gln Val Asp Pro Lys Lys Thr Ile Gln
            20                  25                  30
Met Gly Ser
        35
```

## Description

BACKGROUND OF THE INVENTION

Proliferation and differentiation of cells of multicellular organisms are controlled by hormones and polypeptide growth factors. These diffusable molecules allow cells to communicate with each other and act in concert to form cells and organs, and to repair and regenerate damaged tissue. Examples of hormones and growth factors include the steroid hormones (e.g. estrogen, testosterone), parathyroid hormone, follicle stimulating hormone, the interleukins, platelet derived growth factor (PDGF), epidermal growth factor (EGF), granulocyte-macrophage colony stimulating factor (GM-CSF), erythropoietin (EPO) and calcitonin.

Hormones and growth factors influence cellular metabolism by binding to proteins. Proteins may be integral membrane proteins that are linked to signaling pathways within the cell, such as second messenger systems. Other classes of proteins are soluble molecules, such as the transcription factors.

Thus, there is a continuing need to discover new hormones, growth factors and the like.

SUMMARY OF THE INVENTION

The present invention addresses this need by providing a novel polypeptide and related compositions and methods. Within one aspect, the present invention provides an isolated polynucleotide encoding a mammalian polypeptide termed secretory peptide - 9, hereinafter referred to as Zsig9. The mature human Zsig9 polypeptide is comprised of a sequence of amino acids approximately 64 amino acids long. Amino acid residue 21 of SEQ ID NO: 2, an arginine, is the initial. amino acid of the mature polypeptide. Thus, it is believed that amino residues 1-20 comprise a signal sequence, and the mature Zsig9 polypeptide is represented by the amino acid sequence comprised of residues 21-84. The mature Zsig9 polypeptide is further represented by SEQ ID NO: 3. Within an additional embodiment, the polypeptide further comprises an affinity tag. Within a further embodiment, the polynucleotide is DNA.

Alternative forms of Zsig9 are defined by SEQ ID NOs: 4, 5, and 6. SEQ ID NO: 4 defines a processed form of Zsig9 in which the protein contains amino acid residues 23 - 84 of SEQ ID NO: 2.

SEQ ID NO: 5 represents another form of Zsig9 containing amino acid residues 23, a serine, to and including amino acid 47, a proline of SEQ ID NO: 2.

SEQ ID NO: 6 defines another processed form of Zsig9 contain amino acid residues 50, a threonine, to and including amino acid 84 of SEQ ID NO: 2.

SEQ ID NO: 16 and 17 represent another variant of Zsig9 and SEQ ID NO: 20 represents the mature sequence absent the first 20 amino acid residues, the signal sequence, of SEQ ID NO: 17.

SEQ ID NO: 18 and 19 represent the mouse ortholog of Zsig9; and SEQ ID NO: 21 depicts the mature amino acid sequence absent the first 20 amino acid residues, the signal sequence, of SEQ ID NO: 19.

Within a second aspect of the invention there is provided an expression vector comprising (a) a transcription promoter; (b) a DNA segment encoding a Zsig9 polypeptide as defined by SEQ ID NOs: 2-6, 17, 20 19 and 21 or a polypeptide 90% identical to said polypeptides, and (c) a transcription terminator, wherein the promoter, DNA segment, and terminator are operably linked.

Within a third aspect of the invention there is provided a cultured eukaryotic cell into which has been introduced an expression vector as disclosed above, wherein said cell expresses a protein polypeptide encoded by the DNA segment.

Within a further aspect of the invention there is provided a chimeric polypeptide consisting essentially of a first portion and a second portion joined by a peptide bond. The first portion of the chimeric polypeptide consists essentially of (a) a Zsig9 polypeptide as shown in SEQ ID NOs: 2-6, 17, 20 19 and 21(b) allelic variants of SEQ ID NOs: 2-6, 17, 20 19 and 21; and (c) protein polypeptides that are at least 90% identical to (a) or (b). The second portion of the chimeric polypeptide consists essentially of another polypeptide such as an affinity tag. Within one embodiment the affinity tag is an immunoglobulin $F_c$ polypeptide. The invention also provides expression vectors encoding the chimeric polypeptides and host cells transfected to produce the chimeric polypeptides.

Within an additional aspect of the invention there is provided an antibody that specifically binds to a Zsig9 polypeptide as disclosed above, and also an anti-idiotypic antibody which neutralizes the antibody to a Zsig9 polypeptide.

An additional embodiment of the present invention relates to a peptide or polypeptide which has the amino acid sequence of an epitope-bearing portion of a Zsig9 polypeptide having an amino acid sequence described above. Peptides or polypeptides having the amino acid sequence of an epitope-bearing portion of a Zsig9 polypeptide of the present invention include portions of such polypeptides with at least nine, preferably at least 15 and more preferably at least 30 to 50 amino acids, although epitope-bearing polypeptides of any length up to and including the entire amino acid sequence of a polypeptide of the present invention described above are also included in the present invention.

An example of such a polypeptide is represented by SEQ ID NO:24.

Also claimed are antibodies which bind specifically to the above-defined Zsig9 polypeptides, methods for making said antibodies, and anti-idiotypic antibodies of said Zsig9-binding antibodies.

These and other aspects of the invention will become evident upon reference to the following detailed description.

DETAILED DESCRIPTION OF THE INVENTION

The teachings of all of the references cited herein are incorporated in their entirety herein by reference.

The term "ortholog" (or "species homolog") denotes a polypeptide or protein obtained from one species that has homology to an analogous polypeptide or protein from a different species.

The term "paralog" denotes a polypeptide or protein obtained from a given species that has homology to a distinct polypeptide or protein from that same species.

The term "allelic variant" denotes any of two or more alternative forms of a gene occupying the same chromosomal locus. Allelic variation arises naturally through mutation, and may result in phenotypic polymorphism within populations. Gene mutations can be silent (no change in the encoded polypeptide) or may encode polypeptides having altered amino acid sequence. The term allelic variant is also used herein to denote a protein encoded by an allelic variant of a gene.

The term "expression vector" denotes a DNA molecule, linear or circular, that comprises a segment encoding a polypeptide of interest operably linked to additional segments that provide for its transcription. Such additional segments include promoter and terminator sequences, and may optionally include one or more origins of replication, one or more selectable markers, an enhancer, a polyadenylation signal and the like. Expression vectors are generally derived from plasmid or viral DNA, or may contain elements of both.

The term "isolated", when applied to a polynucleotide, denotes that the polynucleotide has been removed from its natural genetic milieu and is thus free of other extraneous or unwanted coding sequences, and is in a form suitable for use within genetically engineered protein production systems. Such isolated molecules are those that are separated from their natural environment and include cDNA and genomic clones. Isolated DNA molecules of the present invention are free of other genes with which they are ordinarily associated, but may include naturally occurring 5' and 3' untranslated regions such as promoters and terminators. The identification of associated regions will be evident to one of ordinary skill in the art, Dynan and Tijan, *Nature 316:774-78* (1985). When applied to a protein, the term "isolated" indicates that the protein is found in a condition other than its native environment, such as apart from blood and animal tissue. In a preferred form, the isolated protein is substantially free of other proteins, particularly other proteins of animal origin. It is preferred to provide the protein in a highly purified form, i.e., greater than 95% pure, more preferably greater than 99% pure.

The term "operably linked", when referring to DNA segments, denotes that the segments are arranged so that they function in concert for their intended purposes, e.g. transcription initiates in the promoter and proceeds through the coding segment to the terminator.

The term "polynucleotide" is a single- or double-stranded polymer of deoxyribonucleotide or ribonucleotide bases read from the 5' to the 3' end. Polynucleotides include RNA and DNA, and may be isolated from natural sources, synthesized *in vitro,* or prepared from a combination of natural and synthetic molecules.

The term "complements of polynucleotide molecules" denotes polynucleotide molecules having a complementary base sequence and reverse orientation as compared to a reference sequence.

The term "degenerate nucleotide sequence" denotes a sequence of nucleotides that includes one or more degenerate codons (as compared to a reference polynucleotide molecule that encodes a polypeptide). Degenerate codons contain different triplets of nucleotides, but encode the same amino acid residue (i.e., GAU and GAC triplets each encode Asp).

The term "promoter" denotes a portion of a gene containing DNA sequences that provide for the binding of RNA polymerase and initiation of transcription. Promoter sequences are commonly, but not always, found in the 5' non-coding regions of genes.

The term "secretory signal sequence" denotes a DNA sequence that encodes a polypeptide (a "secretory peptide") that, as a component of a larger polypeptide, directs the larger polypeptide through a secretory pathway of a cell in which it is synthesized. The larger peptide is commonly cleaved to remove the secretory peptide during transit through the secretory pathway.

The term "receptor" denotes a cell-associated protein that binds to a bioactive molecule (i.e., a ligand) and mediates the effect of the ligand on the cell. Membrane-bound receptors are characterized by a multi-domain structure comprising an extracellular ligand-binding domain and an intracellular effector domain that is typically involved in signal transduction. Binding of ligand to receptor results in a conformational change in the receptor that causes an interaction between the effector domain and other molecule(s) in the cell. This interaction in turn leads to an alteration in the metabolism of the cell. Metabolic events that are linked to receptor-ligand interactions include gene transcription, phosphorylation, dephosphorylation, increases in cyclic AMP production, mobilization of cellular calcium, mobilization of membrane lipids, cell adhesion, hydrolysis of inositol lipids and hydrolysis of phospholipids. Most nuclear receptors also exhibit a multi-domain structure, including an amino-terminal, transactivating domain, a DNA binding domain and a ligand binding domain. In general, receptors can be membrane bound, cytosolic or nuclear; monomeric (e.g., thyroid stimulating hormone receptor, beta-adrenergic receptor) or multimeric (e.g., PDGF receptor, growth hormone receptor, IL-3 receptor, GM-CSF receptor, G-CSF receptor, erythropoietin receptor and IL-6 receptor).

The term "complement/anti-complement pair" denotes non-identical moieties that form a non-covalently associated, stable pair under appropriate conditions. For instance, biotin and avidin (or streptavidin) are prototypical members of a complement/anti-complement pair. Other exemplary complement/anti-complement pairs include receptor/ligand pairs, antibody/antigen (or hapten or epitope) pairs, sense/antisense polynucleotide pairs, and the like. Where subsequent dissociation of the complement/anti-complement pair is desirable, the complement/anti-complement pair preferably has a binding affinity of $<10^9$ $M^{-1}$.

A "soluble protein" is a protein polypeptide that is not bound to a cell membrane.

Within preferred embodiments of the invention the isolated polynucleotides will hybridize to similar sized regions of polynucleotide defined by SEQ ID NO:1, or a sequence complementary thereto, under stringent conditions. In general, stringent conditions are selected to be about 5°C lower than the thermal melting point ($T_m$) for the specific sequence at a defined ionic strength and pH. The $T_m$ is the temperature (under defined ionic strength and pH) at which 50% of the target sequence hybridizes to a perfectly matched probe. Typical stringent conditions are those in which the salt concentration is about 0.02 M or less at pH 7 and the temperature is at least about 60°C. As previously noted, the isolated polynucleotides of the present invention include DNA and RNA. Methods for isolating DNA and RNA are well known in the art. Total RNA can be prepared using guanidine HCl extraction followed by isolation by centrifugation in a CsCl gradient, Chirgwin *et al., Biochemistry 18*:52-94 (1979). Poly $(A)^+$ RNA is prepared from total RNA using the method of Aviv and Leder, *Proc. Natl. Acad. Sci. USA 69*:1408-1412 (1972). Complementary DNA (cDNA) is prepared from poly$(A)^+$ RNA using known methods. Polynucleotides encoding Zsig9 polypeptides are then identified and isolated by, for example, hybridization or PCR.

The present invention further provides counterpart polypeptides and polynucleotides from other species (orthologs or paralogs). Of particular interest are Zsig9 polypeptides from other mammalian species, including murine, rat, porcine, ovine, bovine, canine, feline, equine and other primate proteins. Species homologs of the human proteins can be cloned using information and compositions provided by the present invention in combination with conventional cloning techniques. For example, a cDNA can be cloned using mRNA obtained from a tissue or cell type that expresses the protein. Suitable sources of mRNA can be identified by probing Northern blots with probes designed from the sequences disclosed ' herein. A library is then prepared from mRNA of a positive tissue of cell line. A Zsig9-encoding cDNA can then be isolated by a variety of methods, such as by probing with a complete or partial human cDNA or with one or more sets of degenerate probes based on the disclosed sequences. A cDNA can also be cloned using the polymerase chain reaction, or PCR (Mullis, U.S. Patent 4,683,202), using primers designed from the sequences disclosed herein. Within an additional method, the cDNA library can be used to transform or transfect host cells, and expression of the cDNA of interest can be detected with an antibody to Zsig9. Similar techniques can also be applied to the isolation of genomic clones.

Those skilled in the art will recognize that the sequences disclosed in SEQ ID NOs:1 and 2, SEQ ID NOs:16 and 17, SEQ ID NOs. 18 and 19, represent a specific alleles of the human Zsig9 gene and polypeptide, and that allelic variation and alternative splicing are expected to occur. Allelic variants can be cloned by probing cDNA or genomic libraries from different individuals according to standard procedures. Allelic variants of the DNA sequence shown in SEQ ID NO: 1, including those containing silent mutations and those in which mutations result in amino acid sequence changes, are within the scope of the present invention, as are proteins which are allelic variants of SEQ ID NOs: 3, 4, 5, and 6.

Additionally, the polynucleotides of the present invention can be synthesized using DNA synthesizers. Currently the method of choice is the phosphoramidite method. Each complementary strand of a double stranded DNA of gene or a gene fragment is made separately. The production of short DNA fragments (60 to 80 bp) can be accomplished by synthesizing the complementary strands and then annealing them. For the production of longer DNA molecules the coupling efficiency of each cycle during chemical DNA synthesis is seldom 100%. To overcome this problem, synthetic genes (double-stranded) are assembled in modular form from single-stranded fragments that are from 20 to 100 nucleotides in length.

One method for building a synthetic gene requires the initial production of a set of overlapping, complementary oligonucleotides, each of which is between 20 to 60 nucleotides long. The sequences of the strands are planned so that, after annealing, the two end segments of the gene are aligned to give blunt ends. Each internal section of the gene has complementary 3' and 5' terminal extensions that are designed to base pair precisely with an adjacent section. Thus, after the gene is assembled, the only remaining requirement to complete the process is sealing the nicks along the backbones of the two strands with T4 DNA ligase. In addition to the protein coding sequence, synthetic genes can be designed with terminal sequences that facilitate insertion into a restriction endonuclease sites of a cloning vector and other sequences should also be added that contain signals for the proper initiation and termination of transcription and translation. See Glick, Bernard R. and Jack J. Pasternak, *Molecular Biotechnology, Principles & Applications of Recombinant DNA*, (ASM Press, Washington, D.C. 1994), Itakura, K. *et al.* Synthesis and use of synthetic oligonucleotides. Annu. *Rev. Biochem. 53* : 323-356 (1984), and Climie, *S. et al.* Chemical synthesis of the thymidylate synthase gene. *Proc. Natl. Acad. Sci. USA 87* :633-637 (1990).

Those skilled in the art will recognize that the sequences disclosed in SEQ ID NOS:1, 2, 3, 4, 5, and 6 represent a single allele of the human. Allelic variants of these sequences can be cloned by probing cDNA or genomic libraries from different individuals according to standard procedures.

The present invention further provides counterpart proteins and polynucleotides from other species ("species orthologs"). Of particular interest are Zsig9 polypeptides from other mammalian species, including murine, porcine, ovine, bovine, canine, feline, equine, and other primates. Species orthologs of the human Zsig9 protein can be cloned using information and compositions provided by the present invention in combination with conventional cloning techniques. For example, a cDNA can be cloned using mRNA obtained from a tissue or cell type that expresses the protein. Suitable sources of mRNA can be identified by probing Northern blots with probes designed from the sequences disclosed herein. A library is then prepared from mRNA of a positive tissue or cell line. A protein-encoding cDNA can then be isolated by a variety of methods, such as by probing with a complete or partial human or mouse cDNA or with one or more sets of degenerate probes based on the disclosed sequences. A cDNA can also be cloned using the polymerase chain reaction, or PCR (Mullis, U.S. Patent No. 4,683,202), using primers designed from the sequences disclosed herein. Within an additional method, the cDNA library can be used to transform or transfect host cells, and expression of the cDNA of interest can be detected with an antibody to the protein. Similar techniques can also be applied to the isolation of genomic clones. As used and claimed the language "an isolated polynucleotide which encodes a polypeptide, said polypeptide being defined by SEQ ID NO: 2-6, 17, 20 19 and 21" includes all allelic variants and species orthologs of the polypeptide of SEQ ID NOs: 2-6, 17, 20 19 and 21.

The present invention also provides isolated protein polypeptides that are substantially homologous to the polypeptides of SEQ ID NOs: 2, 3, 4, 5 or 6 and their species orthologs. By "isolated" is meant a protein or polypeptide that is found in a condition other than its native environment, such as apart from blood and animal tissue. In a preferred form, the isolated polypeptide is substantially free of other polypeptides, particularly other polypeptides of animal origin. It is preferred to provide the polypeptides in a highly purified form, i.e. greater than 95% pure, more preferably greater than 99% pure. The term "substantially homologous" is used herein to denote polypeptides having 50%, preferably 60%, more preferably at least 80%, sequence identity to the sequence shown in SEQ ID NO:2,or its species orthologs. Such polypeptides will more preferably be at least 90% identical, and most preferably 95% or more identical to SEQ ID NO:3,or its species orthologs. Percent sequence identity is determined by conventional methods. See, for example, Altschul *et al., Bull. Math. Bio. 48:* 603-616 (1986) and Henikoff and Henikoff, *Proc. Natl. Acad. Sci. USA 89*: 10915-10919 (1992). Briefly, two amino acid sequences are aligned to optimize the alignment scores using a gap opening penalty of 10, a gap extension penalty of 1, and the "blossom 62" scoring matrix of Henikoff and Henikoff (ibid.) as shown in Table 2 (amino acids are indicated by the standard one-letter codes). The percent identity is then calculated as:

$$\frac{\text{Total number of identical matches}}{\text{[length of the longer sequence plus the number of gaps introduced into the longer sequence in order to align the two sequences]}} \times 100$$

Table 1

| | A | R | N | D | C | Q | E | G | H | I | L | K | M | F | P | S | T | W | Y | V |
|---|---|---|---|---|---|---|---|---|---|---|---|---|---|---|---|---|---|---|---|---|
| A | 4 | | | | | | | | | | | | | | | | | | | |
| R | -1 | 5 | | | | | | | | | | | | | | | | | | |
| N | -2 | 0 | 6 | | | | | | | | | | | | | | | | | |
| D | -2 | -2 | 1 | 6 | | | | | | | | | | | | | | | | |
| C | 0 | -3 | -3 | -3 | 9 | | | | | | | | | | | | | | | |
| Q | -1 | 1 | 0 | 0 | -3 | 5 | | | | | | | | | | | | | | |
| E | -1 | 0 | 0 | 2 | -4 | 2 | 5 | | | | | | | | | | | | | |
| G | 0 | -2 | 0 | -1 | -3 | -2 | -2 | 6 | | | | | | | | | | | | |
| H | -2 | 0 | 1 | -1 | -3 | 0 | 0 | -2 | 8 | | | | | | | | | | | |
| I | -1 | -3 | -3 | -3 | -1 | -3 | -3 | -4 | -3 | 4 | | | | | | | | | | |
| L | -1 | -2 | -3 | -4 | -1 | -2 | -3 | -4 | -3 | 2 | 4 | | | | | | | | | |
| K | -1 | 2 | 0 | -1 | -3 | 1 | 1 | -2 | -1 | -3 | -2 | 5 | | | | | | | | |
| M | -1 | -1 | -2 | -3 | -1 | 0 | -2 | -3 | -2 | 1 | 2 | -1 | 5 | | | | | | | |
| F | -2 | -3 | -3 | -3 | -2 | -3 | -3 | -3 | -1 | 0 | 0 | -3 | 0 | 6 | | | | | | |
| P | -1 | -2 | -2 | -1 | -3 | -1 | -1 | -2 | -2 | -3 | -3 | -1 | -2 | -4 | 7 | | | | | |
| S | 1 | -1 | 1 | 0 | -1 | 0 | 0 | 0 | -1 | -2 | -2 | 0 | -1 | -2 | -1 | 4 | | | | |
| T | 0 | -1 | 0 | -1 | -1 | -1 | -1 | -2 | -2 | -1 | -1 | -1 | -1 | -2 | -1 | 1 | 5 | | | |
| W | -3 | -3 | -4 | -4 | -2 | -2 | -3 | -2 | -2 | -3 | -2 | -3 | -1 | 1 | -4 | -3 | -2 | 11 | | |
| Y | -2 | -2 | -2 | -3 | -2 | -1 | -2 | -3 | 2 | -1 | -1 | -2 | -1 | 3 | -3 | -2 | -2 | 2 | 7 | |
| V | 0 | -3 | -3 | -3 | -1 | -2 | -2 | -3 | -3 | 3 | 1 | -2 | 1 | -1 | -2 | -2 | 0 | -3 | -1 | 4 |

Sequence identity of polynucleotide molecules is determined by similar methods using a ratio as disclosed above.

Substantially homologous proteins and polypeptides are characterized as having one or more amino acid substitutions, deletions or additions. These changes are preferably of a minor nature, that is conservative amino acid substitutions (see Table 3) and other substitutions that do not significantly affect the folding or activity of the protein or polypeptide; small deletions, typically of one to about 30 amino acids; and small amino- or carboxyl-terminal extensions, such as an amino-terminal methionine residue, a small linker peptide of up to about 20-25 residues, or a small extension that facilitates purification (an affinity tag), such as a poly-histidine tract, protein A, Nilsson *et al.*, *EMBO J. 4*:1075, (1985); Nilsson *et al., Methods Enzymol. 198:3,* (1991), glutathione S transferase, Smith and Johnson, *Gene 67*:31, (1988), or other antigenic epitope or binding domain. See, in general Ford *et al.,* Protein Expression and Purification 2: 95-107, (1991). DNAs encoding affinity tags are available from commercial suppliers (e.g., Pharmacia Biotech, Piscataway, NJ).

Table 2

| Conservative amino acid substitutions | |
|---|---|
| Basic | arginine |
| | lysine |
| | histidine |
| Acidic | glutamic acid |
| | aspartic acid |
| Polar | glutamine |
| | asparagine |
| Hydrophobic | leucine |
| | isoleucine |
| | valine |
| Aromatic | phenylalanine |
| | tryptophan |
| | tyrosine |
| Small | glycine |
| | alanine |
| | serine |
| | threonine |
| | methionine |

The proteins of the present invention can also comprise, in addition to the 20 standard amino acids, non-naturally occurring amino acid residues. Non-naturally occurring amino acids include, without limitation, trans-3-methylproline, 2,4-methanoproline, cis-4-hydroxyproline, trans-4-hydroxyproline, N-methyl-glycine, allo-threonine, methylthreonine, hydroxyethyl-cysteine, hydroxyethylhomocysteine, nitroglutamine, homoglutamine, pipecolic acid, tert-leucine, norvaline, 2-azaphenylalanine, 3-azaphenylalanine, 4-azaphenyl-alanine, 4-fluorophenylalanine, 4-hydroxyproline, 6-N-methyl lysine, 2-aminoisobutyric acid, isovaline and $\alpha$-methyl serine. Several methods are known in the art for incorporating non-naturally occurring amino acid residues into proteins. For example, an *in vitro* system can be employed wherein nonsense mutations are suppressed using chemically aminoacylated suppressor tRNAs. Methods for synthesizing amino acids and aminoacylating tRNA are known in the art. Transcription and translation of plasmids containing nonsense mutations are carried out in a cell free system comprising an *E. coli* S30 extract and commercially available enzymes and other reagents. Proteins are purified by chromatography. See, for example, Robertson *et al., J. Am. Chem. Soc. 113*:2722 (1991); Ellman *et al., Meth. Enzymol. 202*:301 (1991); Chung *et al.*, *Science 259*:806-09 (1993); and Chung et *al., Proc. Natl. Acad. Sci. USA 90*:10145-49 (1993). In a second method, translation is carried out in *Xenopus* oocytes by microinjection of mutated mRNA and chemically aminoacylated suppressor tRNAs (Turcatti *et al., J. Biol. Chem. 271*:19991-98 (1996). Within a third method, *E. coli* cells are cultured in the absence of a natural amino acid that is to be replaced (e.g., phenylalanine) and in the presence of the desired non-naturally occurring amino acid(s) (e.g., 2-azaphenylalanine, 3-azaphenylalanine, 4-azaphenylalanine, or 4-fluorophenylalanine). The non-naturally occurring amino acid is incorporated into the protein in place of its natural counterpart. See, Koide *et al., Biochem. 33*:7470-76, 1994. Naturally occurring amino acid residues can be converted to non-naturally occurring species by *in vitro* chemical modification. Chemical modification can be combined with site-directed mutagenesis to further expand the range of substitutions (Wynn and Richards, *Protein Sci. 2*:395-403 (1993).

A limited number of non-conservative amino acids, amino acids that are not encoded by the genetic code, non-naturally occurring amino acids, and unnatural amino acids may be substituted for Zsig9* amino acid residues. "Unnatural amino acids" have been modified after protein synthesis, and/or have a chemical structure in their side chain(s) different from that of the standard amino acids. Unnatural amino acids can be chemically synthesized, or preferably, are commercially available, and include pipecolic acid, thiazolidine carboxylic acid, dehydroproline, 3- and 4-methylproline, and 3,3-dimethylproline.

Essential amino acids in the polypeptides of the present invention can be identified according to procedures known in the art, such as site-directed mutagenesis or alanine-scanning mutagenesis, Cunningham and Wells, *Science 244,* 1081-1085, (1989); Bass *et al., Proc. Natl. Acad. Sci. USA 88*:4498-4502, (1991). In the latter technique, single alanine mutations are introduced at every residue in the molecule, and the resultant mutant molecules are tested for biological activity (e.g., ligand binding and signal transduction) to identify amino acid residues that are critical to the activity of the molecule. Sites of ligand-protein interaction can also be determined by analysis of crystal structure as determined by such techniques as nuclear magnetic resonance, crystallography or photoaffinity labeling. See, for example, de Vos *et al., Science 255*:306-312, (1992); Smith *et al., J. Mol. Biol. 224*:899-904, (1992); Wlodaver et *al., FEBS Lett. 309*:59-64, (1992). The identities of essential amino acids can also be inferred from analysis of homologies with related proteins.

Multiple amino acid substitutions can be made and tested using known methods of mutagenesis and screening, such as those disclosed by Reidhaar-Olson and Sauer, *Science 241*:53-57, (1988) or Bowie and Sauer, *Proc. Natl. Acad. Sci. USA 86*:2152-2156, (1989). Briefly, these authors disclose methods for simultaneously randomizing two or more positions in a polypeptide, selecting for functional polypeptide, and then sequencing the mutagenized polypeptides to determine the spectrum of allowable substitutions at each position. Other methods that can be used include phage display, *e.g*., *Lowman et al., Biochem. 30*:10832-10837, (1991); Ladner *et al.,* U.S. Patent No. 5,223,409; Huse, WIPO Publication WO 92/06204, and region-directed mutagenesis, Derbyshire *et al*., *Gene 46*:145, (1986); Ner *et al., DNA 7*:127, (1988)

Mutagenesis methods as disclosed above can be combined with high-throughput screening methods to detect activity of cloned, mutagenized proteins in host cells. Preferred assays in this regard include cell proliferation assays and biosensor-based ligand-binding assays, which are described below. Mutagenized DNA molecules that encode active proteins or portions thereof (e.g., ligand-binding fragments) can be recovered from the host cells and rapidly sequenced using modern equipment. These methods allow the rapid determination of the importance of individual amino acid residues in a polypeptide of interest, and can be applied to polypeptides of unknown structure.

Using the methods discussed above, one of ordinary skill in the art can prepare a variety of polypeptides that are substantially homologous to SEQ ID NO:2 or allelic variants thereof and retain the properties of the wild-type protein. As expressed and claimed herein the language, "a polypeptide as defined by SEQ ID NO: 2" includes all allelic variants and species orthologs of the polypeptide.

The protein/polypeptides of the present invention, including full-length proteins, protein fragments (e.g. ligand-binding fragments), and fusion polypeptides can be produced in genetically engineered host cells according to conventional techniques. Suitable host cells are those cell types that can be transformed or transfected with exogenous DNA and grown in culture, and include bacteria, fungal cells, and cultured higher eukaryotic cells. Eukaryotic cells, particularly cultured cells of multicellular organisms, are preferred. Techniques for manipulating cloned DNA molecules and introducing exogenous DNA into a variety of host cells are disclosed by Sambrook *et al*., *Molecular Cloning: A Laboratory Manual,* 2nd ed., Cold Spring Harbor Laboratory Press, Cold Spring Harbor, NY, (1989), and Ausubel *et al*., *ibid.*

In general, a DNA sequence encoding a Zsig9 polypeptide is operably linked to other genetic elements required for its expression, generally including a transcription promoter and terminator, within an expression vector. The vector will also commonly contain one or more selectable markers and one or more origins of replication, although those skilled in the art will recognize that within certain systems selectable markers may be provided on separate vectors, and replication of the exogenous DNA may be provided by integration into the host cell genome. Selection of promoters, terminators, selectable markers, vectors and other elements is a matter of routine design within the level of ordinary skill in the art. Many such elements are described in the literature and are available through commercial suppliers.

To direct a Zsig9 polypeptide into the secretory pathway of a host cell, a secretory signal sequence (also known as a leader sequence, prepro sequence or pre sequence) is provided in the expression vector. The secretory signal sequence may be that of the protein, or may be derived from another secreted protein (e.g., t-PA) or synthesized *de novo.* The secretory signal sequence is joined to the Zsig9 DNA sequence in the correct reading frame. Secretory signal sequences are commonly positioned 5' to the DNA sequence encoding the polypeptide of interest, although certain signal sequences may be positioned elsewhere in the DNA sequence of interest (see, *e.g.*, Welch et *al*., U.S. Patent No. 5,037,743; Holland *et al*., U.S. Patent No. 5,143,830).

Cultured mammalian cells are preferred hosts within the present invention. Methods for introducing exogenous DNA into mammalian host cells include calcium phosphate-mediated transfection, Wigler *et al., Cell 14*:725, (1978); Corsaro and Pearson, *Somatic Cell Genetics 7*:603, (1981): Graham and Van der Eb, *Virology 52*:456, (1973), electroporation, Neumann *et al., EMBO J. 1*:841-845, (1982), DEAE-dextran mediated transfection, Ausubel *et al.,* eds., *Current Protocols in Molecular Biology,* John Wiley and Sons, Inc., NY, (1987), and liposome-mediated transfection, Hawley-Nelson *et al., Focus 15*:73, (1993); Ciccarone *et al., Focus 15*:80, (1993). The production of recombinant polypeptides in cultured mammalian cells is disclosed, for example, by Levinson *et al*., U.S. Patent No. 4,713,339; Hagen *et al.,* U.S. Patent No. 4,784,950; Palmiter et *al*., U.S. Patent No. 4,579,821; and Ringold, U.S. Patent No. 4,656,134. Suitable cultured mammalian cells include the COS-1 (ATCC No. CRL 1650), COS-7 (ATCC No. CRL 1651), BHK (ATCC No. CRL 1632), BHK 570 (ATCC No. CRL 10314), 293, ATCC No. CRL 1573; Graham *et al., J. Gen. Virol. 36*:59-72, (1977) and Chinese hamster ovary (e.g. CHO-K1; ATCC No. CCL 61) cell lines. Additional suitable cell lines are known in the art and available from public depositories such as the American Type Culture Collection, Rockville, Maryland. In general, strong transcription promoters are preferred, such as promoters from SV-40 or cytomegalovirus. See, e.g., U.S. Patent No. 4,956,288. Other suitable promoters include those from metallothionein genes (U.S. Patent Nos. 4,579,821 and 4,601,978,and the adenovirus major late promoter.

Drug selection is generally used to select for cultured mammalian cells into which foreign DNA has been inserted. Such cells are commonly referred to as "transfectants". Cells that have been cultured in the presence of the selective agent and are able to pass the gene of interest to their progeny are referred to as "stable transfectants." A preferred selectable marker is a gene encoding resistance to the antibiotic neomycin. Selection is carried out in the presence of a neomycin-type drug, such as G-418 or the like. Selection systems may also be used to increase the expression level of the gene of interest, a process referred to as "amplification." Amplification is carried out by culturing transfectants in the presence of a low level of the selective agent and then increasing the amount of selective agent to select for cells that produce high levels of the products of the introduced genes. A preferred amplifiable selectable marker is dihydrofolate reductase, which confers resistance to methotrexate. Other drug resistance genes (e.g. hygromycin resistance, multi-drug resistance, puromycin acetyltransferase) can also be used.

Other higher eukaryotic cells can also be used as hosts, including insect cells, plant cells and avian cells. Transformation of insect cells and production of foreign polypeptides therein is disclosed by Guarino et al., U.S. Patent No. 5,162,222; Bang et al., U.S. Patent No. 4,775,624; and WIPO publication WO 94/06463. The use of *Agrobacterium rhizogenes* as a vector for expressing genes in plant cells has been reviewed by Sinkar et al., *J. Biosci. (Bangalore) 11*:47-58, (1987).

Fungal cells, including yeast cells, and particularly cells of the genus *Saccharomyces,* can also be used within the present invention, such as for producing protein fragments or polypeptide fusions. Methods for transforming yeast cells with exogenous DNA and producing recombinant polypeptides therefrom are disclosed by, for example, Kawasaki, U.S. Patent No. 4,599,311; Kawasaki et *al*., U.S. Patent No. 4,931,373; Brake, U.S. Patent No. 4,870,008; Welch *et al*., U.S. Patent No. 5,037,743; and Murray *et al*., U.S. Patent No. 4,845,075. Transformed cells are selected by phenotype determined by the selectable marker, commonly drug resistance or the ability to grow in the absence of a particular nutrient (e.g., leucine). A preferred vector system for use in yeast is the *POT1* vector system disclosed by Kawasaki *et al*. (U.S. Patent No. 4,931,373), which allows transformed cells to be selected by growth in glucose-containing media. Suitable promoters and terminators for use in yeast include those from glycolytic enzyme genes (see, e.g., Kawasaki, U.S. Patent No. 4,599,311; Kingsman *et al*., U.S. Patent No. 4,615,974; and Bitter, U.S. Patent No. 4,977,092) and alcohol dehydrogenase genes. See also U.S. Patents Nos. 4,990,446; 5,063,154; 5,139,936 and 4,661,454. Transformation systems for other yeasts, including *Hansenula polymorpha, Schizosaccharomyces pombe, Kluyveromyces lactis, Kluyveromyces fragilis, Ustilago maydis, Pichia pastoris, Pichia methanolica, Pichia guillermondii* and *Candida maltosa* are known in the art. See, for example, Gleeson *et al., J. Gen. Microbiol. 132*:3459-3465, (1986) and Cregg, U.S. Patent No. 4,882,279.

Aspergillus cells may be utilized according to the methods of McKnight *et al*., U.S. Patent No. 4,935,349. Methods for transforming *Acremonium chrysogenum* are disclosed by Sumino *et al.,* U.S. Patent No. 5,162,228. Methods for transforming Neurospora are disclosed by Lambowitz, U.S. Patent No. 4,486,533.

Transformed or transfected host cells are cultured according to conventional procedures in a culture medium containing nutrients and other components required for the growth of the chosen host cells. A variety of suitable media, including defined media and complex media, are known in the art and generally include a carbon source, a nitrogen source, essential amino acids, vitamins and minerals. Media may also contain such components as growth factors or serum, as required. The growth medium will generally select for cells containing the exogenously added DNA by, for example, drug selection or deficiency in an essential nutrient which is complemented by the selectable marker carried on the expression vector or co-transfected into the host cell.

Within one aspect of the present invention, a novel protein is produced by a cultured cell, and the cell is used to screen for a receptor or receptors for the protein, including the natural receptor, as well as agonists and antagonists of the natural ligand.

Proteins of the present invention are useful for enhancing the growth or development of the placenta, heart, and liver. Zsig9 can be measured *in vitro* using cultured cells or *in vivo* by administering molecules of the claimed invention to the appropriate animal model. For instance, Zsig9 transfected (or co-transfected) expression host cells may be embedded in an alginate environment and injected (implanted) into recipient animals. Alginate-poly-L-lysine microencapsulation, permselective membrane encapsulation and diffusion chambers have been described as a means to entrap transfected mammalian cells or primary mammalian cells. These types of non-immunogenic "encapsulations" or microenvironments permit the transfer of nutrients into the microenvironment, and also permit the diffusion of proteins and other macromolecules secreted or released by the captured cells across the environmental barrier to the recipient animal. Most importantly, the capsules or microenvironments mask and shield the foreign, embedded cells from the recipient animal's immune response. Such microenvironments can extend the life of the injected cells from a few hours or days (naked cells) to several weeks (embedded cells).

Alginate threads provide a simple and quick means for generating embedded cells. The materials needed to generate the alginate threads are readily available and relatively inexpensive. Once made, the alginate threads are relatively strong and durable, both *in vitro* and, based on data obtained using the threads, *in vivo*. The alginate threads are easily manipulable and the methodology is scaleable for preparation of numerous threads. In an exemplary procedure, 3% alginate is prepared in sterile $H_2O$, and sterile filtered. Just prior to preparation of alginate threads, the alginate solution is again filtered. An approximately 50% cell suspension (containing about 5 x $10^5$ to about 5 x $10^7$ cells/ml) is mixed with the 3% alginate solution. One ml of the alginate/cell suspension is extruded into a 100 mM sterile filtered $CaCl_2$ solution over a time period of ~15 min, forming a "thread". The extruded thread is then transferred into a solution of 50 mM $CaCl_2$, and then into a solution of 25 mM $CaCl_2$. The thread is then rinsed with deionized water before coating the thread by incubating in a 0.01% solution of poly-L-lysine. Finally, the thread is rinsed with Lactated Ringer's Solution and drawn from solution into a syringe barrel (without needle attached). A large bore needle is then attached to the syringe, and the thread is intraperitoneally injected into a recipient in a minimal volume of the Lactated Ringer's Solution.

An alternative *in vivo* approach for assaying proteins of the present invention involves viral delivery systems. Exemplary viruses for this purpose include adenovirus, herpesvirus, vaccinia virus and adeno-associated virus (AAV). Adenovirus, a double-stranded DNA virus, is currently the best studied gene transfer vector for delivery of heterologous nucleic acid (for a review, see T.C. Becker *et al., Meth. Cell Biol. 43*:161-89 (1994); and J.T. Douglas and D.T. Curiel, *Science & Medicine* 4:44-53 (1997). The adenovirus system offers several advantages: adenovirus can (i) accommodate relatively large DNA inserts; (ii) be grown to high-titer; (iii) infect a broad range of mammalian cell types; and (iv) be used with a large number of available vectors containing different promoters. Also, because adenoviruses are stable in the bloodstream, they can be administered by intravenous injection. Some disadvantages (especially for gene therapy) associated with adenovirus gene delivery include:

(i) very low efficiency integration into the host genome;
(ii) existence in primarily episomal form; and (iii) the host immune response to the administered virus, precluding readministration of the adenoviral vector.

By deleting portions of the adenovirus genome, larger inserts (up to 7 kb) of heterologous DNA can be accommodated. These inserts may be incorporated into the viral DNA by direct ligation or by homologous recombination with a co-transfected plasmid. In an exemplary system, the essential E1 gene has been deleted from the viral vector, and the virus will not replicate unless the E1 gene is provided by the host cell (i.e., the human 293 cell line). When intravenously administered to intact animals, adenovirus primarily targets the liver. If the adenoviral delivery system has an E1 gene deletion, the virus cannot replicate in the host cells. However, the host's tissue (i.e., liver) will express and process (and, if a signal sequence is present, secrete) the heterologous protein. Secreted proteins will enter the circulation in the highly vascularized liver, and effects on the infected animal can be determined.

The adenovirus system can also be used for protein production *in vitro.* By culturing adenovirus-infected non-293 cells under conditions where the cells are not rapidly dividing, the cells can produce proteins for extended periods of time. For instance, BHK cells are grown to confluence in cell factories, then exposed to the adenoviral vector encoding the secreted protein of interest. The cells are then grown under serum-free conditions, which allows infected cells to survive for several weeks without significant cell division. Alternatively, adenovirus vector infected 293S cells can be grown in suspension culture at relatively high cell density to produce significant amounts of protein (see A. Garnier *et al., Cytotechnol. 15*:145-55 (1994). With either protocol, an expressed, secreted heterologous protein can be repeatedly isolated from the cell culture supernatant. Within the infected 293S cell production protocol, non-secreted proteins may also be effectively obtained.

PROTEIN ISOLATION:

Expressed recombinant polypeptides (or chimeric polypeptides) can be purified using fractionation and/or conventional purification methods and media. Ammonium sulfate precipitation and acid or chaotrope extraction may be used for fractionation of samples. Exemplary purification steps may include hydroxyapatite, size exclusion, FPLC and reverse-phase high performance liquid chromatography. Suitable anion exchange media include derivatized dextrans, agarose, cellulose, polyacrylamide, specialty silicas, and the like. PEI, DEAE, QAE and Q derivatives are preferred, with DEAE Fast-Flow Sepharose (Pharmacia, Piscataway, NJ) being particularly preferred. Exemplary chromatographic media include those media derivatized with phenyl, butyl, or octyl groups, such as Phenyl-Sepharose FF (Pharmacia), Toyopearl butyl 650 (Toso Haas, Montgomeryville, PA), Octyl-Sepharose (Pharmacia) and the like; or polyacrylic resins, such as Amberchrom CG 71 (Toso Haas) and the like. Suitable solid supports include glass beads, silica-based resins, cellulosic resins, agarose beads, cross-linked agarose beads, polystyrene beads, cross-linked polyacrylamide resins and the like that are insoluble under the conditions in which they are to be used. These supports may be modified with reactive groups that allow attachment of proteins by amino groups, carboxyl groups, sulfhydryl groups, hydroxyl groups. and/or carbohydrate moieties. Examples of coupling chemistries include cyanogen bromide activation, N-hydroxysuccinimide activation, epoxide activation, sulfhydryl activation, hydrazide activation, and carboxyl and amino derivatives for carbodiimide coupling chemistries. These and other solid media are well known and widely used in the art, and are available from commercial suppliers. Methods for binding receptor polypeptides to support media are well known in the art. Selection of a particular method is a matter of routine design and is determined in part by the properties of the chosen support. See, for example, *Affinity Chromatography: Principles & Methods,* Pharmacia LKB Biotechnology, Uppsala, Sweden, (1988).

The polypeptides of the present invention can be isolated by exploitation of their *properties.* For example, immobilized metal ion adsorption (IMAC) chromatography can be used to purify histidine-rich proteins. Briefly, a gel is first charged with divalent metal ions to form a chelate, E. Sulkowski, *Trends in Biochem. 3*:1-7, (1985). Histidine-rich proteins will be adsorbed to this matrix with differing affinities, depending upon the metal ion used, and will be eluted by competitive elution, lowering the pH, or use of strong chelating agents. Other methods of purification include purification of glycosylated proteins by lectin affinity chromatography and ion exchange chromatography, *Methods in Enzymol.*, Vol. *182,* "Guide to Protein Purification", M. Deutscher, (ed.), Acad. Press, San Diego, (1990), pp.529-39. Alternatively, a fusion of the polypeptide of interest and an affinity tag (e.g., polyhistidine, maltose-binding protein, an immunoglobulin domain) may be constructed to facilitate purification.

Uses

Overexpression of Zsig9 in tumor cells indicates that Zsig9 can be used as an indicator for cancer. Thus, antibodies of Zsig9 can be used as a diagnostic to determine the presence of Zsig9 and thus the presence of cancer as explained below. Furthermore, antibodies labeled with radioisotopes or fused with toxins can be used as a therapeutic to treat cancer. Anti-sense nucleotides derived from the *Zsig9* cDNA can also be used as a therapeutic to inhibit the growth of tumor cells.

Suitable detectable molecules may be directly or indirectly attached to the polypeptide or antibody, and include radionuclides, enzymes, substrates, cofactors, inhibitors, fluorescent markers, chemiluminescent markers, magnetic particles and the like. Suitable cytotoxic molecules may be directly or indirectly attached to the polypeptide or antibody, and include bacterial or plant toxins (for instance, diphtheria toxin, *Pseudomonas* exotoxin, ricin, abrin and the like), as well as therapeutic radionuclides, such as iodine-131, rhenium-188 or yttrium-90 (either directly attached to the polypeptide or antibody, or indirectly attached through means of a chelating moiety, for instance). Polypeptides or antibodies may also be conjugated to cytotoxic drugs, such as adriamycin. For indirect attachment of a detectable or cytotoxic molecule, the detectable or cytotoxic molecule may be conjugated with a member of a complementary/anticomplementary pair, where the other member is bound to the polypeptide or antibody portion. For these purposes, biotin/streptavidin is an exemplary complementary/anticomplementary pair.

In another embodiment, polypeptide-toxin fusion proteins or antibody/fragment-toxin fusion proteins may be used for targeted cell or tissue inhibition or ablation (for instance, to treat cancer cells or tissues). Alternatively, if the polypeptide has multiple functional domains (i.e., an activation domain or a ligand binding domain, plus a targeting domain), a fusion protein including only the targeting domain may be suitable for directing a detectable molecule, a cytotoxic molecule or a complementary molecule to a cell or tissue type of interest. In instances where the domain only fusion protein includes a complementary molecule, the anti-complementary molecule may be conjugated to a detectable or cytotoxic molecule. Such domain-complementary molecule fusion proteins thus represent a generic targeting vehicle for cell/tissue-specific delivery of generic anti-complementary-detectable/ cytotoxic molecule conjugates.

In another embodiment, polypeptide-cytokine fusion proteins or antibody/fragment-cytokine fusion proteins may be used for enhancing *in vitro* cytotoxicity (for instance, that mediated by monoclonal antibodies against tumor targets) and for enhancing *in vivo* killing of target tissues (for example, blood and bone marrow cancers). See, generally, J.L. Hornick *et al.*, Blood 89:4437-47 (1997). In general, cytokines are toxic if administered systemically. The described fusion proteins enable targeting of a cytokine to a desired site of action, thereby providing an elevated local concentration of cytokine. Suitable Zsig9 polypeptides or anti-Zsig9 antibodies target an undesirable cell or tissue (i.e., a tumor or a leukemia), and the fused cytokine mediates improved target cell lysis by effector cells. Suitable cytokines for this purpose include interleukin 2 and granulocyte-macrophage colony-stimulating factor (GM-CSF), for instance.

In yet another embodiment, if the Zsig9 polypeptide or anti-Zsig9 antibody targets vascular cells or tissues, such polypeptide or antibody may be conjugated with a radionuclide, and particularly with a beta-emitting radionuclide, to reduce restenosis. Such therapeutic approach poses less danger to clinicians who-administer the radioactive therapy. For instance, iridium-192 impregnated ribbons placed into stented vessels of patients until the required radiation dose was delivered showed decreased tissue growth in the vessel and greater luminal diameter than the control group, which received placebo ribbons. Further, revascularisation and stent thrombosis were significantly lower in the treatment group. Similar results are predicted with targeting of a bioactive conjugate containing a radionuclide, as described herein.

The bioactive polypeptide or antibody conjugates -described herein can be delivered intravenously, intraarterially or intraductally, or may be introduced locally at the intended site of action.

USES OF POLYNUCLEOTIDE/POLYPEPTIDE:

Molecules of the present invention can be used to identify and isolate receptors of Zsig9. For example, proteins and peptides of the present invention can be immobilized on a column and membrane preparations run over the column (*Immobilized Affinity Ligand Techniques,* Hermanson *et al.,* eds., pp.195-202 (Academic Press, San Diego, CA, 1992). Proteins and peptides can also be radiolabeled *(Methods in Enzymol*., *vol. 182:* "Guide to Protein Purification", M. Deutscher, ed., 721-737 (Acad. Press, San Diego, 1990,) or photoaffinity labeled (Brunner *et al., Ann. Rev. Biochem. 62*:483-514 (1993 and Fedan et *al., Biochem. Pharmacol. 33*:1167-1180 (1984) and specific cell-surface proteins can be identified.

Another aspect of the present invention involves antisense polynucleotide compositions that are complementary to a segment of the polynucleotides set forth in SEQ ID NOs: 1, 16 or 18. Such synthetic antisense oligonucleotides are designed to bind to mRNA encoding Zsig9 polypeptides and to inhibit translation of such mRNA. Such antisense oligonucleotides are used to inhibit expression of Zsig9 polypeptide-encoding genes in cell culture or in a subject.

The present invention also provides reagents which will find use in diagnostic applications. For example, the Zsig9 gene, a probe comprising Zsig9 DNA or RNA or a subsequence thereof can be used to determine if the zZsig9 gene is present on chromosome 12 or if a mutation has -occurred. Detectable chromosomal aberrations at the Zsig9 gene locus include, but are not limited to, aneuploidy, gene copy number changes, insertions, deletions, restriction site changes and rearrangements. Such aberrations can be detected using polynucleotides of the present invention by employing molecular genetic techniques, such as restriction fragment length polymorphism (RFLP) analysis, short tandem repeat (STR) analysis employing PCR techniques, and other genetic linkage analysis techniques known in the art (Sambrook *et al., ibid.;* Ausubel et. al., *ibid.;* A.J. Marian, Chest 108:255-65, 1995).

Transgenic mice, engineered to express the Zsig9 gene, and mice that exhibit a complete absence of Zsig9 gene function, referred to as "knockout mice", Snouwaert *et al., Science 257*:1083 (1992), may also be generated, Lowell *et al., Nature 366*:740-42 (1993). These mice may be employed to study the Zsig9 gene and the protein encoded thereby in an *in vivo* system.

CHROMOSOMAL LOCALIZATION:

Radiation hybrid mapping is a somatic cell genetic technique developed for constructinghigh-resolution, contiguous maps of mammalian chromosomes, Cox *et al., Science 250*:245-50 (1990). Partial or full knowledge of a gene's sequence allows one to design PCR primers suitable for use with chromosomal radiation hybrid mapping panels. Commercially available radiation hybrid mapping panels which cover the entire human genome, such as the Stanford G3 RH Panel and the GeneBridge 4 RH Panel (Research Genetics, Inc., Huntsville, AL), are available. These panels enable rapid, PCR-based chromosomal localizations and ordering of genes, sequence-tagged sites (STSs), and other nonpolymorphic and polymorphic markers within a region of interest. This includes establishing directly proportional physical distances between newly discovered genes of interest and previously mapped markers. The precise knowledge of a gene's position can be useful for a number of purposes, including: 1) determining if a sequence is part of an existing contig and obtaining additional surrounding genetic sequences in various forms, such as YACs, BACs or cDNA clones; 2) providing a possible candidate gene for an inheritable disease which shows linkage to the same chromosomal region; and 3) cross-referencing model organisms, such as mouse, which may aid in determining what function a particular gene might have.

For pharmaceutical use, the proteins of the present invention are formulated for parenteral, particularly intravenous or subcutaneous, delivery according to conventional methods. Intravenous administration will be by bolus injection or infusion over a typical period of one to several hours. In general, pharmaceutical formulations will include a Zsig9 protein in combination with a pharmaceutically acceptable vehicle, such as saline, buffered saline, 5% dextrose in water or the like. Formulations may further include one or more excipients, preservatives, solubilizers, buffering agents, albumin to prevent protein loss on vial surfaces, etc. Methods of formulation are well known in the art and are disclosed, for example, in *Remington's Pharmaceutical Sciences,* 19$^{th}$ Edition Gennaro, ed., (Mack Publishing Co., Easton PA, 1995). Therapeutic doses will generally be in the range of 0.1 to 100 1g/kg of patient weight per day, preferably 0.5-20 1g/kg per day, with the exact dose determined by the clinician according to accepted standards, taking into account the nature and severity of the condition to be treated, patient traits, etc. Determination of dose is within the level of ordinary skill in the art. The proteins may be administered for acute treatment, over one week or less, often over a period of one to three days or may be used in chronic treatment, over several months or years. In general, a therapeutically effective amount of Zsig9 is an amount sufficient to produce a clinically significant change in the liver, kidney, heart or placenta.

Zsig9 Structure

The potential N-terminal 3 dimensional structure of Zsig9 should have similarities to that predicted for amylin and calcitonin-gene related peptides (CGRP). Moving from the N-terminus to the C-terminus there is a predicted disulfide bond between residues 28 and 31 of SEQ ID NO:2. This is. like the predicted disulfide of amylin (positions 35-40) and CGRP1 and 2 (positions 84-89). If the mature form of Zsig9 is not cleaved at the Lys-Lys at position 48-49, there is probably a beta turn followed by an extended region with hydrophobic residues packing along the hydrophobic face of the helix. The C-terminus is probably not amidated as in amylin and CGRP if the mature form is cleaved at the Lys-Lys at position 48-49. If the mature form ends at either the serine at position 81 or at the phenylalanine at position 83, then the glycine at position 82 or the glycine at position 84 may be used to amidate the C-terminus.

Zsig9 Tissue Distribution/Multiple mRNA sizes

Northern analysis shows that Zsig9 is ubiquitous with higher levels in placenta, pancreas, thyroid, prostate, and liver. This may indicate that it is involved in homeostasis. Zsig9 is transcribed by many cell types and is probably released by a variety of cell types in response to some metabolic stress. The Zsig9 receptor is then activated by Zsig9 to alleviate the stress. The stress may include non-optimal levels of sugar, carbohydrate, antigenic load, fat, temperature, pH, $O_2$, osmotic concentration and the like.

Zsig9 is overexpressed in tumor cells. Thus, antibodies to Zsig9 can be used to diagnosis the presence of tumors. Radiolabeled antibodies to Zsig9 can further be used as an imaging agent to locate and treat tumors in the body. Anti-sense nucleotides to *zsig9* can be used to inhibit the progression of tumors.

Therapeutic utility

Cancer Diagnosis and Therapy

As can be seen in Example 5, Zsig9 is overexpressed in a number of human tumors including brain, liver, lung, esophageal, stomach, colon, rectal, thyroid, and lymphoma tumors. Thus, antibodies to Zsig9 can be used both to detect and treat the tumors which overexpress Zsig9. Radiolabeled antibodies to Zsig9 can used both to detect and localize tumors. Antibodies which are either radiolabeled or to which a toxic polypeptide is fused can also be used to treat tumors which overexpress Zsig9.

Nucleotide primers and probes of the *Zsig9* gene can be used to detect the overexpression of Zsig9 using PCR. Gene amplification of *Zsig9* can be determined indirectly by assay of a patient body fluid to detect the presence of elevated levels of Zsig9. Suitable body fluids include serum and urine and exudates of the putative tumor tissues. Examples of immunoassays which can be used in determining the expression of Zsig9 to diagnose neoplastic diseases are described in the patent and scientific literature. See, for example U.S. Patent Nos. 3,791,932; 3,817,837; 3,839,153; 3,850,752; 3,850,578; 3,853,987; 3,867,517; 3,879,262; 3,901,654; 3,935,074; 3,984,533; 3,996,345; 4,034,074; and 4,098,876. Antisense nucleotides to the *Zsig9* DNA and RNA can be administered to a patient to inhibit expression of Zsig9 and thus inhibit tumor growth. Methods for

Antibodies to the Zsig9 polypeptide can be purified and then administered to a patient. These reagents can be combined for therapeutic use with additional active or inert ingredients, *e.g.*, in pharmaceutically acceptable carriers or diluents along with physiologically innocuous stabilizers and excipients. These combinations can be sterile filtered and placed into dosage forms as by lyophilization in dosage vials or storage in stabilized aqueous preparations. This invention also contemplates use of antibodies, binding fragments thereof or single-chain antibodies of the antibodies including forms which are not complement binding.

The quantities of reagents necessary for effective therapy will depend upon many different factors, including means of administration, target site, physiological state of the patient, and other medications administered. Thus, treatment dosages should be titrated to optimize safety and efficacy. Typically, dosages used *in vitro* may provide useful guidance in the amounts useful for *in vivo* administration of these reagents. Animal testing of effective doses for treatment of particular disorders will provide further predictive indication of human dosage. Methods for administration include oral, intravenous, peritoneal, intramuscular, or transdermal administration. Pharmaceutically acceptable carriers will include water, saline, buffers to name just a few. Dosage ranges would ordinarily be expected from 1μg to 1000μg per kilogram of body weight per day. However, the doses by be higher or lower as can be determined by a medical doctor with ordinary skill in the art. For a complete discussion of drug formulations and dosage ranges *see Remington's Pharmaceutical Sciences,* 19th Ed., (Mack Publishing Co., Easton, Penn., 1995), and *Goodman and Gilman's: The Pharmacological Bases of Therapeutics,* 9th Ed. (Pergamon Press 1996).

Nucleic Acid-based Therapeutic Treatment

If a mammal has a mutated or lacks a Zsig9 gene, the Zsig9 gene can be introduced into the cells of the mammal. In one embodiment, a gene encoding a Zsig9 polypeptide is introduced *in vivo* in a viral vector. Such vectors include an attenuated or defective DNA virus, such as but not limited to herpes simplex virus (HSV), papillomavirus, Epstein Barr virus (EBV), adenovirus, adeno-associated virus (AAV), and the like. Defective viruses , which entirely or almost entirely lack viral genes, are preferred. A defective virus is not infective after introduction into a cell. Use of defective viral vectors allows for administration to cells in a specific, localized area, without concern that the vector can infect other cells. Examples of particular vectors include, but are not limited to, a defective herpes virus 1 (HSV1) vector [Kaplitt *et al., Molec. Cell. Neurosci., 2* :320-330 (1991)], an attenuated adenovirus vector, such as the vector described by Stratford-Perricaudet *et al., J. Clin. Invest., 90* :626-630 (1992), and a defective adeno-associated virus vector [Samulski *et al., J. Virol., 61*:3096-3101 (1987); Samulski *et al. J. Virol., 63*:3822-3828 (1989)].

In another embodiment, the gene can be introduced in a retroviral vector, *e.g*., as described in Anderson *et al*., U.S. Patent No. 5,399,346; Mann et *al., Cell,* 33:153 (1983); Temin et *al*., U.S. Patent No. 4,650,764; Temin *et al*., U.S. Patent No. 4,980,289; Markowitz *et al., J. Virol.,* 62:1120 (1988); Temin *et al*., U.S. Patent No. 5,124,263; International Patent Publication No. WO 95/07358, published March 16, 1995 by Dougherty *et al.;* and *Blood, 82:845* (1993).

Alternatively, the vector can be introduced by lipofection *in vivo* using liposomes. Synthetic cationic lipids can be used to prepare liposomes for *in vivo* transfection of a gene encoding a marker [Felgner *et al., Proc. Natl. Acad. Sci. USA, 84*:7413-7417 (1987); see Mackey *et al., Proc. Natl. Acad. Sci. USA,* 85:8027-8031 (1988)]. The use of lipofection to introduce exogenous genes into specific organs *in vivo* has certain practical advantages. Molecular targeting of liposomes to specific cells represents one area of benefit. It is clear that directing transfection to particular cells represents one area of benefit. It is clear that directing transfection to particular cell types would be particularly advantageous in a tissue with cellular heterogeneity, such as the pancreas, liver, kidney, and brain. Lipids may be chemically coupled to other molecules for the purpose of targeting. Targeted peptides, *e.g*., hormones or neurotransmitters, and proteins such as antibodies, or non-peptide molecules could be coupled to liposomes chemically.

It is possible to remove the cells from the body and introduce the vector as a naked DNA plasmid and then re-implant the transformed cells into the body. Naked DNA vector for gene therapy can be introduced into the desired host cells by methods known in the art, *e.g.,* transfection, electroporation, microinjection, transduction, cell fusion, DEAE dextran, calcium phosphate precipitation, use of a gene gun or use of a DNA vector transporter [see, *e.g*., Wu *et al*., *J. Biol. Chem., 267*:963-967 (1992); Wu *et al., J. Biol. Chem*., *263*:14621-14624 (1988)].

Zsig9 polypeptides can also be used to prepare antibodies that specifically bind to Zsig9 epitopes, peptides or polypeptides. The Zsig9 polypeptide or a fragment thereof is inoculate into an animal so as to elicit an immune response. Antibodies generated from this immune response can be isolated and purified as described herein. Methods for preparing and isolating polyclonal and monoclonal antibodies are well known in the art. See, for example, *Current Protocols in Immunology,* Cooligan, *et al*., Eds., (National Institutes of Health, John Wiley and Sons, Inc., 1995); Sambrook *et al., Molecular Cloning: A Laboratory Manual, Second Edition,* (Cold Spring Harbor, NY, 1989); and Hurrell, J. G. R., Ed., *Monoclonal Hybridoma Antibodies: Techniques and Applications*, (CRC Press, Inc., Boca Raton, FL, 1982).

As would be evident to one of ordinary skill in the art, polyclonal antibodies can be generated from inoculating a variety of warm-blooded animals such as horses, cows, goats, sheep, dogs, chickens, rabbits, mice, and rats with a Zsig9 polypeptide or a fragment thereof. The immunogenicity of a Zsig9 polypeptide may be increased through the use of an adjuvant, such as alum (aluminum hydroxide) or Freund's complete or incomplete adjuvant. Polypeptides useful for immunization also include fusion polypeptides, such as fusions of Zsig9 or a portion thereof with an immunoglobulin polypeptide or with maltose binding protein. The polypeptide immunogen may be a full-length molecule or a portion thereof. If the polypeptide portion is "hapten-like", such portion may be advantageously joined or linked to a macromolecular carrier (such as keyhole limpet hemocyanin (KLH), bovine serum albumin (BSA) or tetanus toxoid) for immunization.

As used herein, the term "antibodies" includes polyclonal antibodies, affinity-purified polyclonal antibodies, monoclonal antibodies, and antigen-binding fragments, such as $F(ab')_2$ and Fab proteolytic fragments. Genetically engineered intact antibodies or fragments, such as chimeric antibodies, Fv fragments, single chain antibodies and the like, as well as synthetic antigen-binding peptides and polypeptides, are also included. Non-human antibodies may be humanized by grafting non-human CDRs onto human framework and constant regions, or by incorporating the entire non-human variable domains (optionally "cloaking" them with a human-like surface by replacement of exposed residues, wherein the result is a "veneered" antibody). In some instances, humanized antibodies may retain non-human residues within the human variable region framework domains to enhance proper binding characteristics. Through humanizing antibodies, biological half-life may be increased, and the potential for adverse immune reactions upon administration to humans is reduced.

Alternative techniques for generating or selecting antibodies useful herein include *in vitro* exposure of lymphocytes to Zsig9 protein or peptide, and selection of antibody display libraries in phage or similar vectors (for instance, through use of immobilized or labeled Zsig9 protein or peptide). Genes encoding polypeptides having potential Zsig9 polypeptide binding domains can be obtained by screening random peptide libraries displayed on phage (phage display) or on bacteria, such as *E. coli.* Nucleotide sequences encoding the polypeptides can be obtained in a number of ways, such as through random mutagenesis and random polynucleotide synthesis. These random peptide display libraries can be used to screen for peptides which interact with a known target which can be a protein or polypeptide, such as a ligand or receptor, a biological or synthetic macromolecule, or organic or inorganic substances.

Techniques for creating and screening such random peptide display libraries are known in the art (Ladner et *al*., US Patent NO. 5,223,409; Ladner *et al*., US Patent NO. 4,946,778; Ladner *et al*., US Patent NO. 5,403,484 and Ladner *et al*., US Patent NO. 5,571,698) and random peptide display libraries and kits for screening such libraries are available commercially, for instance from Clontech (Palo Alto, CA), Invitrogen Inc. (San Diego, CA), New England Biolabs, Inc. (Beverly, MA) and Pharmacia LKB Biotechnology Inc. (Piscataway, NJ). Random peptide display libraries can be screened using the Zsig9 sequences disclosed herein to identify proteins which bind to Zsig9. These "binding proteins" which interact with Zsig9 polypeptides can be used for tagging cells; for isolating homolog polypeptides by affinity purification; they can be directly or indirectly conjugated to drugs, toxins, radionuclides and the like. These binding proteins can also be used in analytical methods such as for screening expression libraries and neutralizing activity. The binding proteins can also be used for diagnostic assays for determining circulating levels of polypeptides; for detecting or quantitating soluble polypeptides as marker of underlying pathology or disease. These binding proteins can also act as Zsig9 "antagonists" to block Zsig9 binding and signal transduction in *vitro* and *in vivo.* These anti-Zsig9 binding proteins would be useful for inhibiting the growth of tumors.

Antibodies are determined to be specifically binding if: 1) they exhibit a threshold level of binding activity, and/ or 2) they do not significantly cross-react with related polypeptide molecules. First, antibodies herein specifically bind if they bind to a Zsig9 polypeptide, peptide or epitope with a binding affinity ($K_a$) of $10^6$ $M^{-1}$ or greater, preferably $10^7$ $M^{-1}$ or greater, more preferably $10^8$ $M^{-1}$ or greater, and most preferably $10^9$ $M^{-1}$ or greater. The binding affinity of an antibody can be readily determined by one of ordinary skill in the art, for example, by Scatchard analysis, Scatchard, G., *Ann. NY Acad. Sci. 51*: 660-672 (1949).

Second, antibodies are determined to specifically bind if they do not significantly cross-react with related polypeptides. Antibodies do not significantly cross-react with related polypeptide molecules, for example, if they detect Zsig9 but not known related polypeptides using a standard Western blot analysis (Ausubel *et al., ibid.)*. Examples of known related polypeptides are orthologs, proteins from the same species that are members of a protein family (*e.g*. IL-16), Zsig9 polypeptides, and non-human Zsig9. Moreover, antibodies may be "screened against" known related polypeptides to isolate a population that specifically binds to the inventive polypeptides. For example, antibodies raised to Zsig9 are adsorbed to related polypeptides adhered to insoluble matrix; antibodies specific to Zsig9 will flow through the matrix under the proper buffer conditions. Such screening allows isolation of polyclonal and monoclonal antibodies non-crossreactive to closely related polypeptides, *Antibodies: A Laboratory Manual,* Harlow and Lane (eds.), (Cold Spring Harbor Laboratory Press, 1988); *Current Protocols in Immunology,* Cooligan, *et al.* (eds.), (National Institutes of Health, John Wiley and Sons, Inc., 1995). Screening and isolation of specific antibodies is well known in the art. See, *Fundamental Immunology,* Paul Eds., (Raven Press, 1993); Getzoff *et al., Adv. in Immunol.* 43: 1-98 (1988); *Monoclonal Antibodies:*

*Principles and Practice,* Goding, J.W., Eds., (Academic Press Ltd., 1996); Benjamin *et al., Ann. Rev. Immunol. 2:* 67-101 (1984).

A variety of assays known to those skilled in the art can be utilized to detect antibodies which specifically bind to Zsig9 proteins or peptides. Exemplary assays are described in detail in *Antibodies: A Laboratory Manual,* Harlow and Lane, Eds., (Cold Spring Harbor Laboratory Press, 1988). Representative examples of such assays include: concurrent immunoelectrophoresis, radioimmunoassay, radioimmuno-precipitation, enzyme-linked immunosorbent assay (ELISA), dot blot or Western blot assay, inhibition or competition assay, and sandwich assay. In addition, antibodies can be screened for binding to wild-type versus mutant Zsig9 protein or polypeptide.

Antibodies to Zsig9 may be used for tagging cells that express Zsig9; for isolating Zsig9 by affinity purification; for diagnostic assays for determining circulating levels of Zsig9 polypeptides; for detecting or quantitating soluble Zsig9 as marker of underlying pathology or disease; in analytical methods employing FACS; for screening expression libraries; for generating anti-idiotypic antibodies; and as neutralizing antibodies or as antagonists to block Zsig9 *in vitro* and *in vivo*. Suitable direct tags or labels include radionuclides, enzymes, substrates, cofactors, inhibitors, fluorescent markers, chemiluminescent markers, magnetic particles and the like; indirect tags or labels may feature use of biotin-avidin or other complement/anti-complement pairs as intermediates. Antibodies herein may also be directly or indirectly conjugated to drugs, toxins, radionuclides and the like, and these conjugates used for *in vivo* diagnostic or therapeutic applications. Moreover, antibodies to Zsig9 or fragments thereof may be used *in vitro* to detect denatured Zsig9 or fragments thereof in assays, for example, Western Blots or other assays known in the art.

Another embodiment of the present invention provides for a peptide or polypeptide comprising an epitope-bearing portion of a polypeptide of the invention. The epitope of the this polypeptide portion is an immunogenic or antigenic epitope of a polypeptide of the invention. A region of a protein to which an antibody can bind is defined as an "antigenic epitope". See for instance, Geysen, H.M. *et al., Proc. Natl. Acad Sci. USA 81*:3998-4002 (1984).

As to the selection of peptides or polypeptides bearing an antigenic epitope (*i.e*., that contain a region of a protein molecule to which an antibody can bind), it is well known in the art that relatively short synthetic peptides that mimic part of a protein sequence are routinely capable of eliciting an antiserum that reacts with the partially mimicked protein. See Sutcliffe, J.G. *et al. Science 219*:660-666 (1983). Peptides capable of eliciting protein-reactive sera are frequently represented in the primary sequence of a protein, can be characterized by a set of simple chemical rules, and are confined neither to immunodominant regions of intact proteins (i.e., immunogenic epitopes) nor to the amino or carboxyl terminals. Peptides that are extremely hydrophobic and those of six or fewer residues generally are ineffective at inducing antibodies that bind to the mimicked protein; longer soluble peptides, especially those containing proline residues, usually are effective.

Antigenic epitope-bearing peptides and polypeptides of the invention are therefore useful to raise antibodies, including monoclonal antibodies, that bind specifically to a polypeptide of the invention. Antigenic epitope-bearing peptides and polypeptides of the present invention contain a sequence of at least nine, preferably between 15 to about 30 amino acids contained within the amino acid sequence of a polypeptide of the invention. However, peptides or polypeptides comprising a larger portion of an amino acid sequence of the invention, containing from 30 to 50 amino acids, or any length up to and including the entire amino acid sequence of a polypeptide of the invention, also are useful for inducing antibodies that react with the protein. Preferably, the amino acid sequence of the epitope-bearing peptide is selected to provide substantial solubility in aqueous solvents (*i.e*., the sequence includes relatively hydrophilic residues and hydrophobic residues are preferably avoided); and sequences containing proline residues are particularly preferred. All of the polypeptides shown in the sequence listing contain antigenic epitopes to be used according to the present invention, however, specifically designed antigenic epitopes include the peptides defined by SEQ ID NO:24

BIOACTIVE CONJUGATES:

Antibodies or polypeptides herein can also be directly or indirectly conjugated to drugs, toxins, radionuclides and the like, and these conjugates used for *in vivo* diagnostic or therapeutic applications. For instance, polypeptides or antibodies of the present invention can be used to identify or treat tissues or organs that express a corresponding anti-complementary molecule (receptor or antigen, respectively, for instance). More specifically, Zsig9 polypeptides or anti-Zsig9 antibodies, or bioactive fragments or portions thereof, can be coupled to detectable or cytotoxic molecules and delivered to a mammal having cells, tissues or organs that express the anti-complementary molecule.

Suitable detectable molecules may be directly or indirectly attached to the polypeptide or antibody, and include radionuclides, enzymes, substrates, cofactors, inhibitors, fluorescent markers, chemiluminescent markers, magnetic particles and the like. Suitable cytotoxic molecules may be directly or indirectly attached to the polypeptide or antibody, and include bacterial or plant toxins (for instance, diphtheria toxin, *Pseudomonas* exotoxin, ricin, abrin and the like), as well as therapeutic radionuclides, such as iodine-131, rhenium-188 or yttrium-90 (either directly attached to the polypeptide or antibody, or indirectly attached through means of a chelating moiety, for instance). Polypeptides or antibodies may also be conjugated to cytotoxic drugs, such as adriamycin. For indirect attachment of a detectable or cytotoxic molecule, the detectable or cytotoxic molecule can be conjugated with a member of a complementary/anti-complementary pair, where the other member is bound to the polypeptide or antibody portion. For these purposes, biotin/streptavidin is an exemplary complementary/anticomplementary pair.

In another embodiment, polypeptide-toxin fusion proteins or antibody-toxin fusion proteins can be used for targeted cell or tissue inhibition or ablation (for instance, to treat cancer cells or tissues). Alternatively, if the polypeptide has multiple functional domains (i.e., an activation domain or a ligand binding domain, plus a targeting domain), a fusion protein including only the targeting domain may be suitable for directing a detectable molecule, a cytotoxic molecule or a complementary molecule to a cell or tissue type of interest. In instances where the domain only fusion protein includes a complementary molecule, the anti-complementary molecule can be conjugated to a detectable or cytotoxic molecule. Such domain-complementary molecule fusion proteins thus represent a generic targeting vehicle for cell/tissue-specific delivery of generic anti-complementary-detectable/ cytotoxic molecule conjugates.

In another embodiment, Zsig9-cytokine fusion proteins or antibody-cytokine fusion proteins can be used for enhancing *in vivo* killing of target tissues (for example, blood and bone marrow cancers), if the Zsig9 polypeptide or anti-Zsig9 antibody targets the hyperproliferative blood or bone marrow cell (See, generally, Hornick *et al., Blood 89*:

4437-47 (1997). They described fusion proteins enable targeting of a cytokine to a desired site of action, thereby providing an elevated local concentration of cytokine. Suitable Zsig9 polypeptides or anti-Zsig9 antibodies target an undesirable cell or tissue (i.e., a tumor or a leukemia), and the fused cytokine mediated improved target cell lysis by effector cells. Suitable cytokines for this purpose include interleukin-2 and granulocyte-macrophage colony-stimulating factor (GM-CSF), for instance.

In yet another embodiment, if the Zsig9 polypeptide or anti- Zsig9 antibody targets vascular cells or tissues, such polypeptide or antibody may be conjugated with a radionuclide, and particularly with a beta-emitting radionuclide, to reduce restenosis or to reduce the growth of blood vessels in tumors. Such therapeutic approach poses less danger to clinicians who administer the radioactive therapy.

The bioactive polypeptide or antibody conjugates described herein can be delivered intravenously, intraarterially or intraductally, or may be introduced locally at the intended site of action.

USES OF POLYNUCLEOTIDE/POLYPEPTIDE:

Molecules of the present invention can be used to identify and isolate receptors involved in the binding of Zsig9. For example, proteins and peptides of the present invention can be immobilized on a column and membrane preparations run over the column, *Immobilized Affinity Ligand Techniques,* Hermanson *et al*., eds., pp.195-202 (Academic Press, San Diego, CA, 1992). Proteins and peptides can also be radiolabeled *Methods in Enzymol., vol. 182,* "Guide to Protein Purification", M. Deutscher, ed., p.p. 721-737 (Acad. Press, San Diego, 1990) or photoaffinity labeled, Brunner *et al., Ann. Rev. Biochem. 62*:483-514 1993 and Fedan *et al., Biochem. Pharmacol. 33*:1167-80 (1984) and specific cell-surface proteins can be identified.

GENE THERAPY:

Polynucleotides encoding Zsig9 polypeptides are useful within gene therapy applications where it is desired to increase or inhibit Zsig9 activity. If a mammal has a mutated or absent Zsig9 gene, the Zsig9 gene can be introduced into the cells of the mammal. In one embodiment, a gene encoding a Zsig9 polypeptide is introduced *in vivo* in a viral vector. Such vectors include an attenuated or defective DNA virus, such as, but not limited to, herpes simplex virus (HSV), papillomavirus, Epstein Barr virus (EBV), adenovirus, adeno-associated virus (AAV), and the like. Defective viruses, which entirely or almost entirely lack viral genes, are preferred. A defective virus is not infective after introduction into a cell. Use of defective viral vectors allows for administration to cells in a specific, localized area, without concern that the vector can infect other cells. Examples of particular vectors include, but are not limited to, a defective herpes simplex virus 1 (HSV1) vector (Kaplitt *et al., Molec. Cell. Neurosci.* 2:320-30 (1991); an attenuated adenovirus vector, such as the vector described by Stratford-Perricaudet *et al., J. Clin. Invest. 90*:626-30 (1992); and a defective adeno-associated virus vector, Samulski *et al., J. Virol. 61*:3096-101 (1987); Samulski *et al., J. Virol. 63*:3822-8 (1989).

In another embodiment, a Zsig9 gene can be introduced in a retroviral vector, *e.g.,* as described in Anderson *et al*., U.S. Patent No. 5,399,346; Mann *et al. Cell 33*:153 (1983); Temin *et al.,* U.S. Patent No. 4,650,764; Temin *et al.,* U.S. Patent No. 4,980,289; Markowitz *et al., J. Virol. 62*:1120 (1988); Temin *et al*., U.S. Patent No. 5,124,263; International Patent Publication No. WO 95/07358, published March 16, 1995 by Dougherty *et al.*; and Kuo *et al., Blood 82*:845 (1993). Alternatively, the vector can be introduced by lipofection *in vivo* using liposomes. Synthetic cationic lipids can be used to prepare liposomes for *in vivo* transfection of a gene encoding a marker (Felgner *et al., Proc. Natl. Acad. Sci. USA 84*:7413-7 (1987); Mackey *et al., Proc. Natl. Acad. Sci. USA 85*:8027-31 (1988). The use of lipofection to introduce exogenous genes into specific organs *in vivo* has certain practical advantages. Molecular targeting of liposomes to specific cells represents one area of benefit. More particularly, directing transfection to particular cells represents one area of benefit. For instance, directing transfection to particular cell types would be particularly advantageous in a tissue with cellular heterogeneity, such as the pancreas, liver, kidney, and brain. Lipids may be chemically coupled to other molecules for the purpose of targeting. Targeted peptides (e.g., hormones or neurotransmitters), proteins such as antibodies, or non-peptide molecules can be coupled to liposomes chemically.

It is possible to remove the target cells from the body; to introduce the vector as a naked DNA plasmid; and then to re-implant the transformed cells into the body. Naked DNA vectors for gene therapy can be introduced into the desired host cells by methods known in the art, *e.g.,* transfection, electroporation, microinjection, transduction, cell fusion, DEAE dextran, calcium phosphate precipitation, use of a gene gun or use of a DNA vector transporter. See, *e. g.,* Wu *et al., J. Biol. Chem. 267*:963-7 (1992); Wu *et al*., *J. Biol. Chem. 263*:14621-4 (1988).

Antisense methodology can be used to inhibit Zsig9 gene transcription, such as to inhibit cell proliferation in vivo. Polynucleotides that are complementary to a segment of a Zsig9-encoding polynucleotide (*e.g.*, a polynucleotide as set froth in SEQ ID NO:1) are designed to bind to Zsig9-encoding mRNA and to inhibit translation of such mRNA. Such antisense polynucleotides are used to inhibit expression of Zsig9 polypeptide-encoding genes in cell culture or in a subject.

The present invention also provides reagents which will find use in diagnostic applications. For example, the Zsig9 gene, a probe comprising Zsig9 DNA or RNA or a subsequence thereof can be used to determine if the Zsig9 gene is present on chromosome 12 or if a mutation has occurred. Detectable chromosomal aberrations at the Zsig9 gene locus include, but are not limited to, aneuploidy, gene copy number changes, insertions, deletions, restriction site changes and rearrangements. Such aberrations can be detected using polynucleotides of the present invention by employing molecular genetic techniques, such as restriction fragment length polymorphism (RFLP) analysis, short tandem repeat (STR) analysis employing PCR techniques, and other genetic linkage analysis techniques known in the art (Sambrook et *al., ibid.;* Ausubel et. al., *ibid.;* Marian, *Chest 108*:255-65 (1995).

The invention is further illustrated by the following non-limiting examples.

Example 1. Cloning of Zsig9

Zsig9 was identified from expressed sequence tag (EST) SEQ ID NO: 7. The cDNA clone containing the EST was discovered in a placenta from a full-term pregnancy cDNA library which contained the EST. The cDNA was isolated from *E. coli* transfected with the plasmid and then streaked out on an LB 100 µg/ml ampicillin and 100 µg/ml methicillin plate. The cDNA insert was sequenced. The insert was determined to be 649 base pairs long with a 84 amino acid open reading frame and a putative 20 amino acid signal peptide.

Example 2

Construction of Zsig9 Expression Vectors

Two Zsig9 construction vectors were made in a flag amino acid sequence (SEQ ID NO: 8) was inserted onto the N-terminal or C-terminal ends of the Zsig9 polypeptide. For the construction in which the flag amino acid sequence was attached to the N-terminus of Zsig9, a 473 bp Zsig9 PCR DNA fragment was generated with 1 µl of a ¼ dilution of the plasmid prep of Example 1 and 1 microliter (µl) of SEQ ID NO: 9 and 10 each having a concentration of 20 picomoles (pm)/µl of primer. The PCR mixture contained 2.5 µl of 10X PCR buffer,0.5 KLENTAQ (both from CLONTECH), 2.5 µl REDI-LOAD dye (Research Genetics), 2.5 nucleotide triphosphate mix (Perkin-Elmer) and 14 µl of water. The PCR reaction was incubated at 94°C for 5 minutes, and then run for 10 cycles each individual cycle being comprised of 30 seconds at 94°C and 2 minutes at 75°C. This was followed by 15 cycles each cycle being comprised of 30 seconds at 94°C and 2 minutes at 60°C. The reaction was ended with an incubation for 10 minutes at 74°C.

The resultant PCR mixture was then run on a 0.9% LMP agarose gel with TBE buffer. After the gel was run the band containing the DNA was cut out and the DNA was purified from the gel with a QUIAQUICK® column (Qiagen). 100 µl of the DNA was digested in a solution containing 4 µl of B buffer, 1 µl of *BamH*1 (Boehringer Mannheim) and 1 µl of *XhoI* (Boehringer Mannheim) for 2 hours at 37°C. The digested reaction mixture was electrophoresed on a 1% TBE gel; the DNA band was excised with a razor blade and the DNA was extracted from the gel with the Qiaquick® Gel Extraction Kit (Qiagen).

The excised DNA was subcloned into plasmid NF/pZP9 which had been cut with *Bam* and *Xho*. NF/pZP9 is a mammalian cell expression vector comprising an expression cassette containing the mouse metallothionein-1 promoter, a sequence encoding the tissue plasminogen activator (TPA) leader, then the flag peptide (SEQ ID NO:8), then multiple restriction sites. These were followed by the human growth hormone terminator, an *E. coli* origin of replication and a mammalian selectable marker expression unit containing the SV40 promoter, enhancer and origin of replication; a dihydrofolate reductase gene (DHFR) and the SV40 terminator. 1 µl containing 10 ng of the NF/pZP9 vector which had been previously digested with *Xho* and *Bam*HI was mixed with 1 µl of 10X ligase buffer, 1 µl of T4 ligase and 2 µl of Zsig9 fragment containing 20 ng. The ligation took place at room temperature for 3 hours and then electroporated into DH10b cells. After the electroporation the cells were plated onto LB-amp plates.

For the construction of the Zsig9 gene in which the flag polypeptide SEQ ID NO: 8 was inserted onto the C-terminus of the Zsig9 polypeptide, a 649 bp Zsig9 PCR fragment was generated with 1 µl of ¼ dilution of the plasmid preparation containing Zsig9 described in Example 1 and 20 pm each of primers SEQ ID NO: 11 and SEQ ID NO: 12. The PCR reaction was incubated at 94°C for 5 minutes, then run for 10 cycles, each cycle being comprised 30 seconds at 94°C and 2 minutes at 75°C. This was followed by 15 cycles each cycle comprised of 30 seconds at 94°C and 2 minutes at 60°C. The reaction was ended with a final 10 minute extension at 74°C.

The entire reaction mixture was run on a 1% TBE gel and the DNA was cut out with a razor blade and the DNA was extracted using the QIAQUICK™ gel extraction kit. 20 µl out of the recovered 35 µl digested with 10 units of BamHI (Boehringer Mannheim) and 10 units of *Eco*R1(Gibco BRL) for 2 hours at 37°C. The digested PCR mixture was electrophoresed on a 1% TBE gel. The DNA band was cut out with a razor blade and the DNA was extracted from the gel using the QIAquick® Gel Extraction Kit (Qiagen). The extracted DNA was subcloned into plasmid CF/pZP9 which had been cut with *Eco*R1 and *Bam*H1. Plasmid cfpzp9 is a mammalian expression vector containing an expression cassette having the mouse metallothionein-1 promoter, multiple restriction sites for insertion of coding sequences, a sequence encoding the flag peptide, SEQ ID NO:10, a stop codon, a human growth hormone terminator, an *E. coli* origin of replication, a mammalian selectable marker expression unit having an SV40 promoter, enhancer and origin of replication, a DHFR gene and the SV40 terminator.

Example 3

Tissue Distribution

Human Multiple Tissue Northern Blots (MTN I, MTN II, and MTN III; Clontech) were probed to determine the tissue distribution of human ZSIG-9 expression. A 40 bp probe (SEQ ID NO: 13) was used to probe the blots. The 5' end of the probe was radioactively labeled using T4 polynucleotide kinase and forward reaction buffer (GIBCO BRL, Gaithersburg, MD) according to the manufacturer's specifications. The probe was purified using a NUCTRAP push column (Stratagene, La Jolla, CA). ExpressHyb™ (Clontech) solution was used for prehybridization and as a hybridizing solution for the Northern blots.
Hybridization took place overnight at 42°C using 2 x $10^6$ cpm/ml of labeled probe. The blots were then washed at 55°C in 1X SSC, 0.1% SDS. A 1.2 kb transcript was detected. The signal was strongest in heart, placenta, liver and kidney. An intermediate signal was detected in spleen, thymus, prostate, testis, ovary, small intestine, colon, peripheral blood lymphocytes, thyroid, spinal cord. Weak signal was detected in lymph node, trachea, adrenal gland and bone marrow.

EXAMPLE 4

Chromosomal Assignment and Placement of Zsig9.

Zsig9 was mapped to chromosome 12 using the commercially available "GeneBridge 4 Radiation Hybrid Panel" (Research Genetics, Inc., Huntsville, AL). The GeneBridge 4 Radiation Hybrid Panel contains PCRable DNAs from each of 93 radiation hybrid clones, plus two control DNAs (the HFL donor and the A23 recipient). A publicly available WWW server (http://www-genome.wi.mit.edu/cgi-bin/contig/rhmapper.pl) allows mapping relative to the Whitehead Institute/MIT Center for Genome Research's radiation hybrid map of the human genome (the "WICGR" radiation hybrid map) which was constructed with the GeneBridge 4 Radiation Hybrid Panel.

For the mapping of Zsig9 with the "GeneBridge 4 RH Panel", 20 µl reactions were set up in a PCRable 96-well microtiter plate (Stratagene, La Jolla, CA) and used in a "RoboCycler Gradient 96" thermal cycler (Stratagene). Each of the 95 PCR reactions consisted of 2 µl 10X KlenTaq PCR reaction buffer (CLONTECH Laboratories, Inc., Palo Alto, CA), 1.6 µl dNTPs mix (2.5 mM each, PERKIN-ELMER, Foster City, CA), 1 µl sense primer, SEQ ID NO: 14, 1 µl antisense primer, SEQ ID NO: 15, 2 µl "RediLoad"
(Research Genetics, Inc., Huntsville, AL), 0.4 µl 50X Advantage KlenTaq Polymerase Mix (Clontech Laboratories, Inc.), 25 ng of DNA from an individual hybrid clone or control and x µl $ddH_2O$ for a total volume of 20 µl. The reactions were overlaid with an equal amount of mineral oil and sealed. The PCR cycler conditions were as follows: an initial 1 cycle 5 minute denaturation at 95°C, 35 cycles of a 1 minute denaturation at 95°C, 1 minute annealing at 62°C and 1.5 minute extension at 72°C, followed by a final 1 cycle extension of 7 minutes at 72°C. The reactions were separated by electrophoresis on a 3% NuSieve GTG agarose gel (FMC Bioproducts, Rockland, ME).

The results showed that Zsig9 maps 344.72 cR_3000 from the top of the human chromosome 12 linkage group on the WICGR radiation hybrid map. Proximal and distal framework markers were WI-6672 (D12S1410) and RP_L41_1, respectively. The use of the surrounding markers positions Zsig9 in the 12q15 region on the integrated LDB chromosome 12 map (The Genetic Location Database, University of Southhampton, WWW server: http://cedar. genetics. soton.ac.uk/public_html/).

Example 5

Northern Blot Analysis of Human Tumors

Northern Analysis was carried out on the following Tumor Blots ; Human Tumor Panel Blot I, Human Tumor Panel Blot II, Human Tumor Panel Blot V, Human Stomach Tumor Blot, and Human Colon Tumor Blot (Clontech, Palo Alto, California). A probe was obtained using a PCR product representing the full length coding sequence of zsig9. The probe was radioactively labeled with 32P using Rediprime Labeling System from Amersham (England). The probe was purified using a NUCTRAP push column(Stratagene Cloning Systems, La Jolla, Ca.) . EXPRESSHYB (Clontech, Palo Alto, Ca.) solution was used for prehybridization and hybridization. The hybridization solution consisted of 8 mls EXPRESSHYB, 80 µl Sheared Salmon Sperm DNA (10mg/ml,5 Prime-3 Prime, Boulder, CO) , 48 µl Human Cot-1 DNA (1mg/ml, Gibco BRL) and 20 µl labeled probe (8 x 10-5 CPM/µl). Hybridization took place overnight at 55°C And the blots were then washed in 2X SSC,0.1%SDS at RT, then 2X SSC,0.1% SDS at 60°C, followed by 0.1X SSC, 0.1% SDS wash at 60°C. The blots were exposed overnight and developed.

A transcript of .8-lkb was observed in the following -tissues. The strongest signals were in brain tumor, liver tumor, esophageal tumor, stomach tumor, colon tumor, rectal tumor, and thyroid tumor. Weaker signals were in adrenal tumor and normal adrenal, peratoid tumor, and lymphoma tumor. Weakest signals were observed in normal liver, normal esophagus, normal stomach, normal colon, normal rectum, normal thyroid, and normal lymphoma. The Stomach and Colon Tumor Blots showed signals consistent with those observed in the panel blots for stomach and colon tissue.

Example 6

Chromosomal Assignment and Placement of murine Zsig9

Murine Zsig9 was mapped in mouse to chromosome 10 using the commercially available mouse T31 whole genome radiation hybrid (WGRH) panel (Research Genetics, Inc., Huntsville, AL) and the Map Manager QT linkage analysis program. At P = 0.0001, murine Zsig9 linked proximal to D10Mit136 at 62.0 cM with a LOD score of 4.3. This is a known region of synteny or linkage conservation with regions of human chromosome 12.

The T31 WGRH panel contains PCRable DNAs from each of 100 radiation hybrid clones, plus two control DNAs (the 129aa donor and the A23 recipient). For the mapping of murine Zsig9 with the T31 WGRH panel, 20 µl reactions were set up in PCRable 96-well microtiter plates (Stratagene, La Jolla, CA) and used in "RoboCycler Gradient 96" thermal cyclers (Stratagene). Each of the 102 PCR reactions consisted of 2 µl 10X KlenTaq PCR reaction buffer (CLONTECH Laboratories, Inc., Palo Alto, CA), 1.6 µl dNTPs mix (2.5 mM each, PERKIN-ELMER, Foster City, CA), 1 µl sense primer, (SEQ ID NO:22), 5' TCG CGC GAG AGT TTG GAG 3', 1 µl antisense primer, (SEQ ID NO:23), 5' CCC AGC TTC CCG CAC TTA 3', 2 µl "RediLoad" (Research Genetics, Inc., Huntsville, AL), 0.4 µl 50X Advantage KlenTaq Polymerase Mix (Clontech Laboratories, Inc.), 25 ng of DNA from an individual hybrid clone or control and x µl ddH2O for a total volume of 20 µl. The reactions were overlaid with an equal amount of mineral oil and sealed. The PCR cycler conditions were as follows: an initial 1 cycle 5 minute denaturation at 94°C, 35 cycles of a 1 minute denaturation at 94°C, 1 minute annealing at 62°C and 1.5 minute extension at 72°C, followed by a final 1 cycle extension of 7 minutes at 72°C. The reactions were separated by electrophoresis on a 2% agarose gel (Life Technologies, Gaithersburg, MD). The use of the surrounding markers positions murine Zsig9 in the 51-62 centiMorgan (cM) region on mouse chromosome 10 map. Other genes of interest which lie in or near this region are glioma-associated oncogene homolog, myogenic factors 5 and 6, mast cell growth factor, insulin-like growth factor, and tumor rejection antigen-1.

Certain particular aspects and embodiments of the present invention are illustrated by the following clauses:

1. An isolated polynucleotide which encodes a mammalian polypeptide selected from the group consisting of SEQ ID NOs:17, 19, 20 and 21 or a polypeptide which is at least 90% identical to the polypeptides of said group.

2. An expression vector comprising the following operably linked elements:

   a transcription promoter;
   a polynucleotide which encodes a mammalian polypeptide selected from the group consisting of SEQ ID NOs: 17, 19, 20 and 21 or a polypeptide which is at least 90% identical to the polypeptides of said group; and a transcription terminator.

3. An isolated polypeptide selected from the group of amino acid sequences consisting of SEQ ID NOs: 2-6 or a polypeptide which is at least 90% identical to said polypeptides.

4. An isolated polypeptide selected from the group of amino acid sequences consisting of SEQ ID NOs: 17, 20 19 and 21 or a polypeptide which is at least 90% identical to said polypeptides.

5. An antibody, antibody fragment or single-chain antibody that specifically binds to a mammalian polypeptide, said polypeptide being defined by the amino acid sequences of SEQ ID NOs: 2-6.

6. An antibody, antibody fragment or single-chain antibody that specifically binds to a mammalian polypeptide, said polypeptide being defined by the amino acid sequences of SEQ ID NOs: 17, 20 19 and 21.

7. An antibody of Clauses 5 and 6 wherein said antibody is either monoclonal or polyclonal.

8. The antibody, antibody fragment or single-chain antibody of Clauses 5-7 wherein said antibody, antibody fragment or single-chain antibody is humanized.

9. A method for producing an antibody which binds to a mammalian polypeptide or a peptide or polypeptide defined by SEQ ID NOs: 2-6 or to a peptide or polypeptide which is at least 90% identical to said peptide or polypeptide comprising bringing into contact a polypeptide defined by SEQ ID NOs: 2-6 or to a peptide or polypeptide which is at least 90% identical to said peptide or polypeptide with a cell capable or producing antibodies or the cell is brought into contact with a nucleic acid which encodes said peptide or polypeptide, wherein said cell produces antibodies to said peptide or polypeptide; and
isolating said antibody.

10. A method for producing an antibody which binds to a polypeptide defined by SEQ ID NOs: 17, 19, 20 and 21 or to a peptide or polypeptide which is at least 90% identical to said peptide or polypeptide comprising bringing into contact a polypeptide defined by SEQ ID NOs: 17, 20 19 and 21 or to a peptide or polypeptide which is at least 90% identical to said peptide or polypeptide with a cell capable or producing antibodies or the cell is brought into contact with a nucleic acid which encodes said peptide or polypeptide, wherein said cell produces antibodies to said peptide or polypeptide; and
isolating said antibody.

11. The method of Clauses 9 or 10 wherein an animal is inoculated with the peptide or polypeptide or nucleic acid under conditions wherein the animal produces antibodies to said peptide; and isolating said antibodies

12. The method of Clauses 9, 10 or 11 wherein the antibodies are polyclonal or monoclonal.

13. An anti-idiotypic antibody, anti-idiotypic antibody fragment or anti-idiotypic single-chain antibody which binds to an antibody, an antibody fragment or single-chain antibody of a polypeptide defined by SEQ ID NOs: 2-6 or to a polypeptide which is at least 90% identical to said polypeptide.

14. An anti-idiotypic antibody, anti-idiotypic antibody fragment or anti-idiotypic single-chain antibody which binds to an antibody, an antibody fragment or single-chain antibody of a polypeptide defined by SEQ ID NOs: 17, 19, 20 and 21 or to a polypeptide which is at least 90% identical to said polypeptide.

## Claims

1. An antibody, antibody fragment or single chain antibody that specifically binds to a mammalian Zsig9 polypeptide defined by SEQ IDNOS: 2-6, 17, 19, 20 or 21 or to a polypeptide that is at least 90% identical to said polypeptide.

2. An antibody, antibody fragment or single chain antibody according to claim 1, further comprising a detectable marker.

3. An antibody, antibody fragment or single chain antibody according to claim 2, wherein the detectable marker is selected from a radionuclide; an enzyme; a substrate; a cofactor; an inhibitor; a fluorescent marker; a chemiluminescent marker; or a magnetic particle.

4. An anti-idiotypic antibody, anti-idiotypic antibody fragment or anti-idiotypic single chain antibody which binds to an antibody, antibody fragment or single chain antibody that specifically binds to a mammalian Zsig9 polypeptide defined by SEQ ID NOS: 2-6, 17, 19, 20 or 21 or to a polypeptide that is at least 90% identical to said polypeptide.

5. Use of an antibody, antibody fragment or single chain antibody that binds to a human Zsig9 polypeptide selected from the group consisting of SEQ ID NOS: 2-6, 17 and 20, in the manufacture of a composition for in vitro diagnosis of cancer in a sample of human tissue.

6. Use of an antibody, antibody fragment or single chain antibody that binds to a mouse Zsig9 polypeptide selected from the group consisting of SEQ ID NOS: 19 and 21, in the manufacture of a composition for in vitro diagnosis of cancer in a sample of human tissue.

**7.** Use according to claims 5 or 6, wherein the antibody, antibody fragment or single chain antibody is monoclonal.

**8.** Use of a polynucleotide which encodes a mammalian Zsig9 polypeptide selected from the group consisting of SEQ ID NOS: 2-6, 17, 19, 20 and 21, or a polypeptide which is at least 90% identical to the polypeptides of said group, in the manufacture of a composition for in vitro diagnosis of cancer in a sample of human tissue.

**9.** Use of an antisense polynucleotide that is complementary to a Zsig9 polynucleotide selected from the group consisting of SEQ ID NOS: 1, 16 or 18, or a segment thereof, in the manufacture of a composition for in vitro diagnosis of cancer in a sample of human tissue.

**10.** Use according to claim 9, wherein the antisense polynucleotide is a probe comprising either a DNA or an RNA sequence.

**11.** Use according to any of claims 5-10, wherein the cancer is selected from brain tumour, liver tumour, lung tumour, esophageal tumour, stomach tumour, colon tumour, rectal tumour, thyroid tumour, or lymphoma tumour.

**12.** An in vitro method of diagnosing cancer in a sample of human tissue comprising determining the expression of Zsig9 in said sample.

**13.** A method according to claim 12, wherein the sample is solid tissue comprising all or a part of a tumour.

**14.** A method according to claim 12, wherein the sample comprises patient body fluid.

**15.** A method according to claim 14, wherein the patient body fluid is selected from serum, urine or exudates of putative tumour tissues.

**16.** A method according to any of claims 12-15, wherein Zsig9 expression is determined by binding of an antibody to a Zsig9 mammalian polypeptide present in the sample, wherein said Zsig9 polypeptide is selected from the group consisting of SEQ ID NOS: 2-6, 17, 19, 20 or 21 or a polypeptide that is at least 90% identical to the polypeptides of said group.

**17.** A method according to claim 16, wherein the antibody is monoclonal.

**18.** A method according to any of claims 12-15, wherein Zsig9 expression is determined by binding of an antisense polynucleotide to a Zsig9 polynucleotide present in the sample, wherein the Zsig9 polynucleotide is selected from the group consisting of SEQ ID NOS: 1, 16 or 18, or a segment thereof.

**19.** A method according to claim 18, wherein the antisense polynucleotide is a probe comprising either a DNA or an RNA sequence.